(19) Europäisches Patentamt
European Patent Office
Office européen des brevets

(11) Veröffentlichungsnummer: **0 375 791**
**A1**

(12) # EUROPÄISCHE PATENTANMELDUNG

(21) Anmeldenummer: **88119850.1**

(22) Anmeldetag: **29.11.88**

(51) Int. Cl.⁵: **C07C 215/30, C07C 229/38, C07C 215/54, C07C 217/62, C07C 255/59, C07C 215/68, C07C 225/16**

(43) Veröffentlichungstag der Anmeldung:
**04.07.90 Patentblatt 90/27**

(84) Benannte Vertragsstaaten:
**ES GR**

(71) Anmelder: **BOEHRINGER INGELHEIM VETMEDICA GMBH**

**D-6507 Ingelheim/Rhein(DE)**

(72) Erfinder: **Hurnaus, Rudolf, Dr. Dipl.-Chem.**
**Silcherstrasse 19**
**D-7950 Biberach 1(DE)**

Erfinder: **Reiffen, Manfred, Dr. Dipl.-Chem.**
**Matthias-Erzberger-Strasse 40**
**D-7950 Biberach 1(DE)**
Erfinder: **Sauter, Robert, Dr. Dipl.-Chem.**
**Albert-Schweitzer-Weg 9**
**D-7958 Laupheim(DE)**
Erfinder: **Grell, Wolfgang, Dr. Dipl.-Chem.**
**Geschwister-Scholl-Strasse 18**
**D-7950 Biberach 1(DE)**
Erfinder: **Rupprecht, Eckhard, Dr.**
**Riedbachstrasse 15**
**D-7960 Aulendorf-Tannhausen(DE)**

(54) **Neue Phenylethanolamine, deren Verwendung als Arzneimittel und als Leistungsförderer bei Tieren sowie Verfahren zu ihrer Herstellung.**

(57) Die Erfindung betrifft neue Phenylethanolamine der allgemeinen Formel

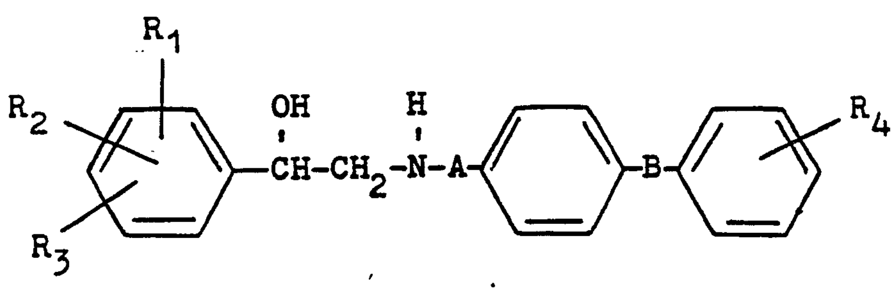

,(I)

in der
A eine geradkettige oder verzweigte Alkylengruppe,
B eine Bindung, eine Alkylengruppe, eine Carbonyl- oder Hydroxymethylengruppe,
$R_1$ ein Wasserstoffatom, ein Halogenatom oder eine Trifluormethylgruppe,
$R_2$ ein Wasserstoffatom oder eine Aminogruppe,
$R_3$ eine Cyanogruppe, ein Wasserstoff-, Chlor- oder Bromatom und
$R_4$ ein Wasserstoff- oder Halogenatom, eine Alkyl-, Hydroxy-, Alkoxy-, Carboxy-, Alkoxycarbonyl-, Aminocarbonyl-, Alkylaminocarbonyl- oder Dialkylaminocarbonylgruppe oder eine Alkoxygruppe, die endständig durch eine Carboxy-, Alkoxycarbonyl-, Aminocarbonyl-, Alkylaminocarbonyl-, Dialkylaminocarbonyl-, Hydroxy-, Alkoxy-, Amino-, Alkylamino-, Dialkylamino-, Pyrrolidino-, Piperidino- oder Hexamethylenimino-

EP 0 375 791 A1

gruppe substituiert ist, bedeuten, deren optische Isomere und deren Diastereomere sowie deren Säureadditionssalze.

Die neuen Verbindungen der Formel I, deren optische Isomere und deren Diastereomere sowie deren Säureadditionssalze, insbesondere deren physiologisch verträgliche Säureadditionssalze mit anorganischen oder organischen Säuren, weisen wertvolle pharmakologische Eigenschaften auf, nämlich eine Wirkung auf den Stoffwechsel, vorzugsweise eine blutzuckersenkende und körperfettreduzierende Wirkung, außerdem können diese als Leistungsförderer bei Tieren eingesetzt werden.

Die neuen Verbindungen der obigen allgemeinen Formel I lassen sich nach an und für sich bekannten Verfahren herstellen.

# Neue Phenylethanolamine, deren Verwendung als Arzneimittel und als Leistungsförderer bei Tieren sowie Verfahren zu ihrer Herstellung

Die vorliegende Erfindung betrifft neue Phenylethanolamine der Formel

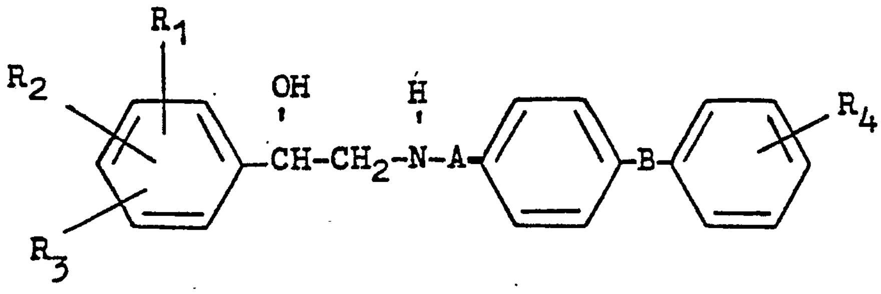

,(I)

deren optische Isomere, Diastereomere und deren Additionssalze, insbesondere für die pharmazeutische Verwendung deren physiologisch verträgliche Additionssalze, welche wertvolle pharmakologische Eigenschaften aufweisen, nämlich eine Wirkung auf den Stoffwechsel, insbesondere eine blutzuckersenkende, körperfettreduzierende und Energieverbrauch-steigernde Wirkung, sowie eine Senkung der atherogenen Lipoproteine VLDL und LDL.

Außerdem können die neuen Verbindungen als Leistungsförderer bei Tieren, insbesondere zur Erzielung höherer täglicher Gewichtszunahmen und verbesserter Futterausnutzung in der Tierernährung, vorzugsweise in der Tiermast, eingesetzt werden, was ein weiterer Gegenstand der vorliegenden Erfindung ist.

In der obigen allgemeinen Formel I bedeuten

A eine geradkettige oder verzweigte Alkylengruppe mit 1 bis 5 Kohlenstoffatomen,

B eine Bindung, eine Alkylengruppe mit 1 oder 2 Kohlenstoffatomen, eine Carbonyl- oder Hydroxymethylengruppe,

$R_1$ ein Wasserstoffatom, ein Halogenatom oder eine Trifluormethylgruppe,

$R_2$ ein Wasserstoffatom oder eine Aminogruppe,

$R_3$ eine Cyanogruppe, ein Wasserstoff-, Chlor- oder Bromatom und

$R_4$ ein Wasserstoff- oder Halogenatom, eine Alkyl-, Hydroxy-, Alkoxy-, Carboxy-, Alkoxycarbonyl-, Aminocarbonyl-, Alkylaminocarbonyl- oder Dialkylaminocarbonylgruppe, eine Alkoxygruppe mit 1 bis 3 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, oder eine Alkoxygruppe mit 2 oder 3 Kohlenstoffatomen, die endständig durch eine Hydroxy-, Alkoxy-, Amino-, Alkylamino-, Dialkylamino-, Pyrrolidino-, Piperidino- oder Hexamethyleniminogruppe substituiert ist, wobei alle vorstehend erwähnten Alkyl- oder Alkoxygruppen, sofern nichts anderes erwähnt wurde, jeweils 1 bis 3 Kohlenstoffatome enthalten können.

Für die bei der Definition der Reste eingangs erwähnten Bedeutungen kommt beispielsweise

für A die der Methylen-, 1-Ethyliden-, 1-n-Propyliden-, 1-n-Butyliden-, Ethylen-, 1-Methyl-ethylen-, 2-Methyl-ethylen-, 1-Ethyl-ethylen-, 2-Ethyl-ethylen-, 1,2-Dimethyl-ethylen-, 1,1-Dimethyl-ethylen-, 1,1-Diethyl-ethylen-, 1-Ethyl-1-methyl-ethylen-, 2,2-Dimethyl-ethylen-, 2,2-Diethyl-ethylen-, 2-Ethyl-2-methyl-ethylen-, n-Propylen-, 1-Methyl-n-propylen-, 2-Methyl-n-propylen-, 3-Methyl-n-propylen-, 1-Ethyl-n-propylen-, 2-Ethyl-n-propylen-, 3-Ethyl-n-propylen, 1,1-Dimethyl-n-propylen-, 2,3-Dimethyl-n-propylen-, 3,3-Dimethyl-n-propylen-, n-Butylen- oder n-Pentylengruppe, für B die der Bindung, der Methylen-, Ethylen-, 1-Ethyliden-, Carbonyl- oder Hydroxymethylengruppe,

für $R_1$ die des Wasserstoff-, Fluor-, Chlor- oder Bromatoms oder der Trifluormethylgruppe,

für $R_2$ die des Wasserstoffatoms oder der Aminogruppe, für $R_3$ die des Wasserstoff-, Chlor- oder Bromatoms oder der Cyangruppe und

für $R_4$ die des Wasserstoff-, Fluor-, Chlor- oder Bromatoms, der Methyl-, Ethyl-, n-Propyl-, Isopropyl-, Hydroxy-, Methoxy-, Ethoxy-, n-Propoxy-, Isopropoxy-, n-Butoxy-, Carboxy-, Methoxycarbonyl-, Ethoxycarbonyl-, n-Propoxycarbonyl-, Aminocarbonyl-, Methylaminocarbonyl-, Ethylaminocarbonyl-, Isopropylaminocarbonyl-, Dimethylaminocarbonyl-, Diethylaminocarbonyl-, Di-n-propylaminocarbonyl-, N-Ethyl-methylaminocarbonyl-, N-Ethyl-isopropylaminocarbonyl-, 2-Hydroxyethoxy-, 3-Hydroxy-n-propoxy-, 2-Methoxy-ethoxy-, 2-Ethoxyethoxy-, 2-n-Propoxy-ethoxy-, 3-Ethoxy-n-propoxy-, 2-Aminoethoxy-, 2-Methylamino-ethoxy-, 2-Dimethylamino-ethoxy-, 2-Isopropylamino-ethoxy-, 2-Di-n-propylamino-ethoxy-, 2-(1-Pyrrolidino)ethoxy-, 2-(1-Piperidino)ethoxy-, 2-(1-Hexamethylenimino)ethoxy-, 3-Amino-n-propoxy-, 3-Diethylamino-n-propoxy-, 3-(1-Piperidino)-n-propoxy-, Carboxymethoxy-, 2-Carboxy-ethoxy-, 3-Carboxy-n-propoxy-, Methoxycarbonylmethoxy-, 2-Methoxycarbonyl-ethoxy-, Ethoxycarbonylmethoxy-, 2-Ethoxycarbonyl-ethoxy-, 3-Ethoxycarbonyl-n-propoxy-, n-Propoxycarbonylmethoxy-, 2-Isopropoxycarbonyl-ethoxy-, Aminocarbonylmethoxy-, 2-Aminocarbonyl-ethoxy-, Methylaminocarbonylmethoxy-, 2-Methylaminocarbonyl-ethoxy-, Dimethylaminocarbonylmethoxy-, 2-Dimethylaminocarbonyl-ethoxy-, Diethylaminocarbonylmethoxy-, 2-Diethylaminocarbonylethoxy- oder 2-Di-n-propylaminocarbonyl-ethoxygruppe in Betracht.

Beispielsweise seien zusätzlich zu den in den Beispielen genannten Verbindungen noch folgende Verbindungen genannt, die unter die vorstehend erwähnte allgemeine Formel I fallen:

4′-[2-[N-(2-(4-Amino-3-cyano-5-fluor-phenyl)-2-hydroxy-ethyl)amino]propyl]diphenylmethan-2-carbonsäure-ethylester,
4′-[2-[N-(2-(4-Amino-3-cyano-5-fluor-phenyl)-2-hydroxy-ethyl)amino]propyl]biphenylyl-4-carbonsäure-ethylester,
4′-[2-[N-(2-(4-Amino-3-cyano-5-fluor-phenyl)-2-hydroxy-ethyl)amino]propyl]biphenylyl-4-oxyessigsäure-ethylester,
4′-[2-[N-(2-(4-Amino-3-cyano-5-fluor-phenyl)-2-hydroxy-ethyl)amino]propyl]biphenylyl-2-carbonsäure-ethylester,
4′-[2-[N-(2-(3-Chlor-phenyl)-2-hydroxy-ethyl)amino]propyl]biphenylyl-4-oxyessigsäure-amid und
4′-[2-[N-(2-(3-Chlor-phenyl)-2-hydroxy-ethyl)amino]propyl]-4-[2-(2-ethoxy)ethoxy]biphenyl,
deren optische Isomere, Diastereomere und Additionssalze, insbesondere deren physiologisch verträgliche Additionssalze.

Bevorzugte Verbindungen der Formel I sind jedoch diejenigen, in denen

A eine gegebenenfalls durch eine Alkylgruppe mit 1 bis 3 Kohlenstoffatomen oder durch zwei Methylgruppen substituierte Ethylengruppe,
B eine Bindung, eine Methylen-, Ethylen-, Hydroxymethylen-oder Carbonylgruppe,
$R_1$ ein Wasserstoff-, Fluor-, Chlor- oder Bromatom oder eine Trifluormethylgruppe,
$R_2$ ein Wasserstoffatom oder eine Aminogruppe,
$R_3$ ein Wasserstoffatom, ein Chloratom oder eine Cyanogruppe und
$R_4$ ein Wasserstoffatom, ein Chloratom, eine Hydroxy-, Methoxy-, Methyl-, Ethyl-, Carboxy-, Methoxycarbonyl-, Ethoxycarbonyl-, Carboxymethoxy-, 2-Hydroxy-ethoxy-, Methoxycarbonylmethoxy- oder Ethoxycarbonylmethoxygruppe bedeuten, deren optische Isomere, Diastereomere und Additionssalze, insbesondere deren physiologisch verträgliche Additionssalze.

Besonders bevorzugte Verbindungen der vorliegenden Erfindung sind jedoch die Verbindungen der allgemeinen Formel

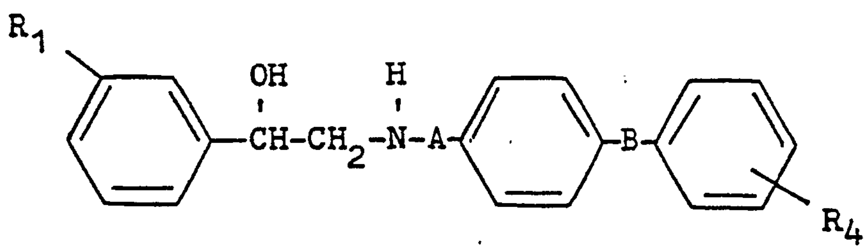
,(Ia)

in der
A eine Ethylen- oder Methyl-ethylengruppe,
B eine Bindung oder eine Methylengruppe,
$R_1$ ein Wasserstoff- oder Chloratom und
$R_4$ ein Wasserstoffatom, eine Methyl-, Ethyl-, Hydroxy-, Methoxy-, Methoxycarbonyl-, Ethoxycarbonyl-, 2-Hydroxy-ethoxy-, Methoxycarbonylmethoxy- oder Ethoxycarbonylmethoxygruppe in 2- oder 4-Stellung bedeuten, deren optische Isomere, Diastereomere und Additionssalze, insbesondere deren physiologisch verträgliche Additionssalze.

Erfindungsgemäß erhält man die neuen Verbindungen nach folgenden Verfahren:

a) Umsetzung einer Verbindung der allgemeinen Formel

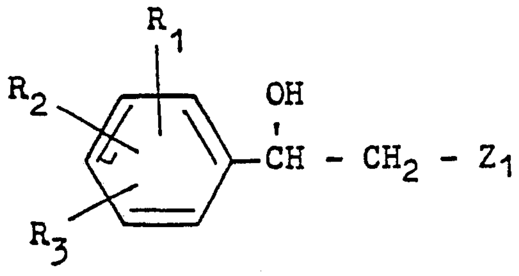

,(II)

mit einer Verbindung der allgemeinen Formel

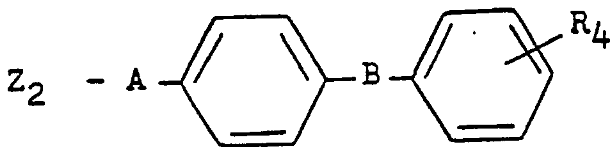

,(III)

in denen
A, B und $R_1$ bis $R_4$ wie eingangs definiert sind, einer der Reste $Z_1$ oder $Z_2$ eine nukleophile Austrittsgruppe und
der andere der Reste $Z_1$ oder $Z_2$ eine Aminogruppe bedeuten.

Als nukleophile Austrittsgruppen kommen beispielsweise Halogenatome oder Sulfonyloxygruppen, z.B. ein Chlor-, Bromoder Jodatom, die Methansulfonyloxy-, p-Toluolsulfonyloxyoder Ethoxysulfonyloxygruppe, in Betracht.

Die Umsetzung wird zweckmäßigerweise in einem Lösungsmittel oder Lösungsmittelgemisch wie Aceton, Diethylether, Acetonitril, Dimethylformamid, Dimethylsulfoxid, Methylenchlorid, Chloroform, Tetrahydrofuran, Dioxan oder in einem Überschuß der eingesetzten Verbindungen der allgemeinen Formeln II und/oder III und gegebenenfalls in Gegenwart eines säurebin denden Mittels, z.B. eines Alkoholats wie Kalium-tert•butylat, eines Alkalihydroxids wie Natrium- oder Kaliumhydroxid, eines Alkalicarbonats wie Kaliumcarbonat, einer tertiären organischen Base wie Triethylamin, N,N-Diisopropylethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes, zweckmäßigerweise bei Temperaturen zwischen 0 und 150°C, vorzugsweise bei Temperaturen zwischen 20 und 100°C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel II oder III durchgeführt.

b) Reduktion einer gegebenenfalls im Reaktionsgemisch gebildeten Schiff'schen Base der allgemeinen Formel

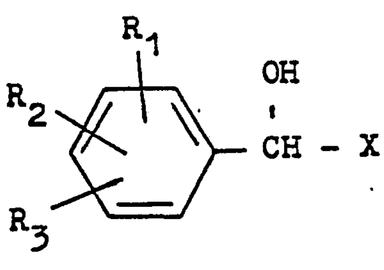

,(IV)

in der
$R_1$ bis $R_3$ wie eingangs definiert sind und
X eine Gruppe der Formel

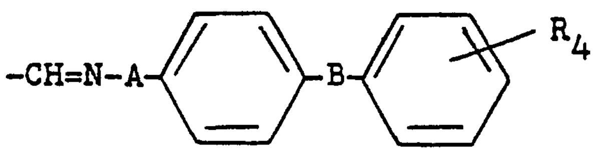

oder

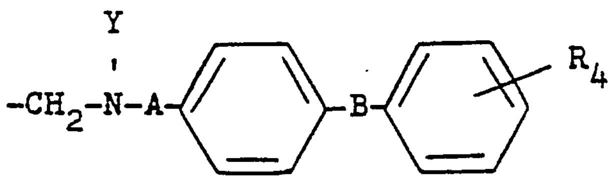

darstellt, wobei

A, B und $R_4$ wie eingangs definiert sind und

Y zusammen mit einem Wasserstoffatom des benachbarten Kohlenstoffatomes des Restes A eine weitere Bindung bedeutet.

Die Reduktion wird in einem geeigneten Lösungsmittel wie Methanol, Ethanol, Diethylether, Tetrahydrofuran, Dioxan, Essigsäure-ethylester oder Ethanol/Essigsäure-ethylester mit Wasserstoff in Gegenwart eines Hydrierungskatalysators wie Platin, Palladium/Kohle oder Raney-Nickel bei einem Wasserstoffdruck von 1 bis 5 bar oder mit einem Metallhydrid wie Lithiumaluminiumhydrid, Diboran, Natriumcyanborhydrid oder Boran/Dimethylsulfid, vorzugsweise jedoch mit Natriumborhydrid oder Natriumcyanborhydrid bei Temperaturen zwischen 0 und 50° C, vorzugsweise bei Raumtemperatur, durchgeführt. Bei der Reduktion mit einem komplexen Metallhydrid wie Lithiumaluminiumhydrid, Diboran oder Boran/Dimethylsulfid, kann eine vorhandene Carbonyl- oder Nitrilfunktion mitreduziert werden.

c) Reduktion einer gegebenenfalls im Reaktionsgemisch gebildeten Verbindung der allgemeinen Formel

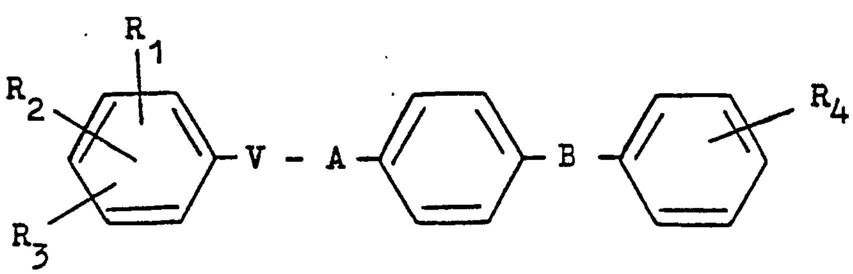

,(V)

in der A, B und $R_1$ bis $R_4$ wie eingangs definiert sind und

V eine Gruppe der Formel

$-CO-CH_2-\overset{H}{N}-$ oder

$-CO-CH=N-$ darstellt.

Die Reduktion wird vorzugsweise in einem geeigneten Lösungsmittel wie Methanol, Ethanol, Diethylether oder Tetrahydrofuran in Gegenwart eines Metallhydrids wie Natriumborhydrid, Lithiumaluminiumhydrid, Diboran, Boran/Dimethylsulfid oder Natriumcyanborhydrid, vorzugsweise jedoch mit Natriumborhydrid in Methanol oder Ethanol, zwischen 0 und 40° C, vorzugsweise jedoch bei Raumtemperatur, durchgeführt.

Bei der Reduktion mit einem komplexen Metallhydrid wie Natriumborhydrid, Lithiumaluminiumhydrid, Diboran oder Boran/Dimethylsulfid, kann eine vorhandene Carbonyl- oder Nitrilfunktion mitreduziert werden.

d) Umsetzung einer Verbindung der allgemeinen Formel

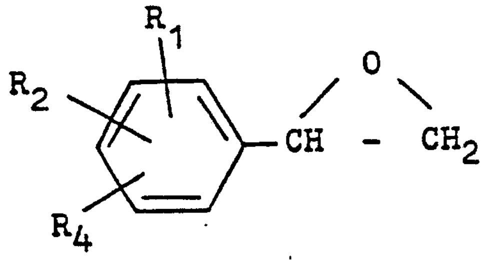

,(VI)

in der
$R_1$ bis $R_3$ wie eingangs definiert sind, mit einem Amin der allgemeinen Formel

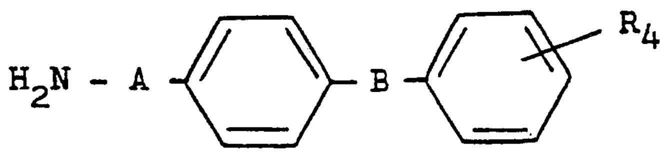

,(VII)

in der
A, B und $R_4$ wie eingangs definiert sind.

Die Umsetzung wird vorzugsweise in einem Lösungsmittel wie Ethanol, Isopropanol, Tetrahydrofuran, Dioxan, Dimethylformamid, Dimethylsulfoxid oder Acetonitril/Ethanol zweckmäßigerweise bei Temperaturen zwischen 50 und 100 °C durchgeführt. Die Umsetzung kann jedoch auch ohne Lösungsmittel durchgeführt werden.

e) Reduktion einer gegebenenfalls im Reaktionsgemisch gebildeten Verbindung der allgemeinen Formel

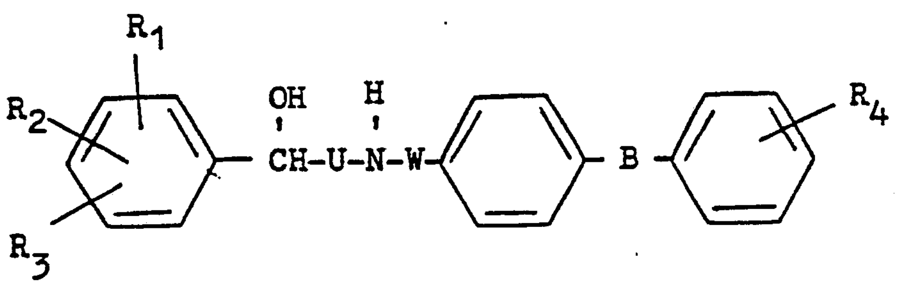

,(VIII)

in der
B und $R_1$ bis $R_4$ wie eingangs definiert sind,
U eine Carbonylgruppe und W die für A eingangs erwähnten Bedeutungen besitzt oder
U eine Methylengruppe und W eine Alkanongruppe mit 1 bis 5 Kohlenstoffatomen darstellt, wobei die Carbonylgruppe mit dem Stickstoffatom der NH-Gruppe verknüpft sein muß.

Die Reduktion wird in einem geeigneten Lösungsmittel wie Diethylether oder Tetrahydrofuran mit einem Reduktionsmittel wie einem Metallhydrid, z.B. mit Lithiumaluminiumhydrid, Diboran oder Diboran/Dimethylsulfid, vorzugsweise jedoch mit Natriumborhydrid in Gegenwart von Eisessig oder Trifluoressigsäure, bei Temperaturen zwischen 0 und 100 °C, vorzugs weise jedoch bei Temperaturen zwischen 10 und 50 °C, durchgeführt. - Bei der Reduktion wird eine gegebenenfalls vorhandene Carbonyl- oder Nitrilfunktion gleichzeitig mitreduziert.

f) Zur Herstellung von Verbindungen der allgemeinen Formel I, in der $R_3$ ein Wasserstoff-, Chlor- oder Bromatom und $R_4$ eine Alkoxygruppe mit 2 oder 3 Kohlenstoffatomen, die endständig durch eine Hydroxy-, Alkoxy-, Amino-, Alkylamino-, Dialkylamino-, Pyrrolidino-, Piperidino- oder Hexamethyleniminogruppe substituiert ist, darstellen:

Reduktion einer Verbindung der allgemeinen Formel

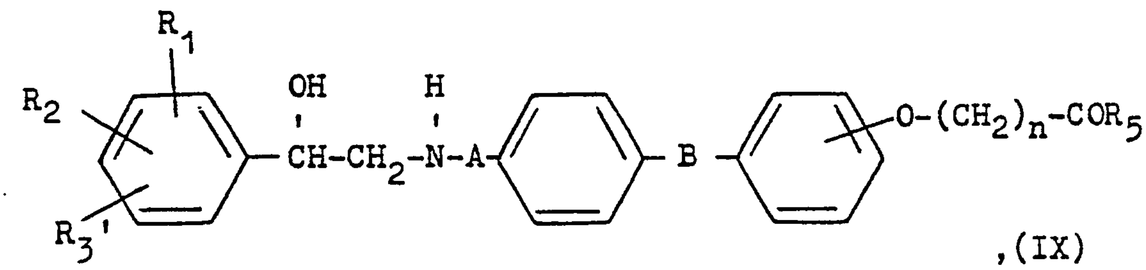

,(IX)

in der

A, B, $R_1$ und $R_2$ wie eingangs definiert sind,

$R_3'$ ein Wasserstoff-, Chlor- oder Bromatom, n die Zahl 1 oder 2 und

$R_5$ eine Hydroxy-, Alkoxy-, Amino-, Alkylamino-, Dialkylamino-, Pyrrolidino-, Piperidino- oder Hexamethyleniminogruppe darstellen.

Die Reduktion wird in einem geeigneten Lösungsmittel wie Diethylether oder Tetrahydrofuran mit einem Reduktionsmittel wie einem Metallhydrid, z.B. mit Lithiumaluminiumhydrid, Diboran, Diboran/Dimethylsulfid oder mit Natriumborhydrid in Gegenwart von Eisessig oder Trifluoressigsäure, bei Temperaturen zwischen 0 und 100 °C, vorzugsweise jedoch bei Temperaturen zwischen 10 und 50 °C, durchgeführt. - Bei der Umsetzung kann eine im Rest B enthaltene Carbonylgruppe gleichzeitig mitreduziert werden.

g) Zur Herstellung von Verbindungen der allgemeinen Formel I, in der $R_4$ eine Alkoxycarbonyl-, Aminocarbonyl-, Alkylaminocarbonyl- oder Dialkylaminocarbonylgruppe, eine Alkoxygruppe mit 1 bis 3 Kohlenstoffatomen, die endständig durch eine Alkoxycarbonyl-, Aminocarbonyl-, Alkylaminocarbonyl-oder Dialkylaminocarbonylgruppe substituiert ist, darstellt:

Umsetzung einer Verbindung der allgemeinen Formel

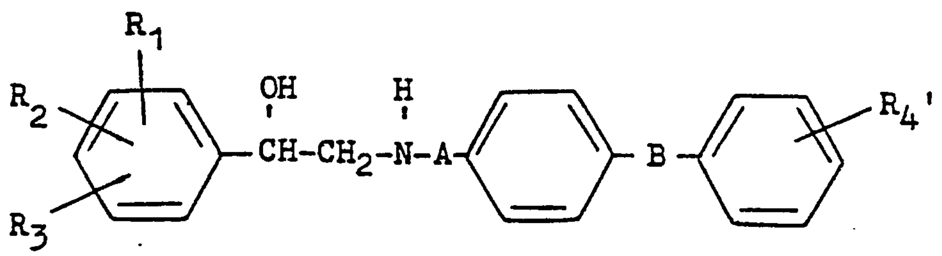

,(X)

in der

A, B und $R_1$ bis $R_3$ wie eingangs definiert sind und

$R_4'$ eine Carboxygruppe oder eine Alkoxygruppe mit 1 bis 3 Kohlenstoffatomen, welche endständig durch eine Carboxygruppe substituiert ist, darstellt, oder deren reaktionsfähige Derivate wie beispielsweise deren aktivierte Ester mit einer Verbindung der allgemeinen Formel

$H - R_6$ ,(XI)

in der

$R_6$ eine Alkoxy-, Amino-, Alkylamino-, Dialkylamino-, Pyrrolidino-, Piperidino- oder Hexamethyleniminogruppe darstellt, wobei jeweils der Alkyl- oder Alkoxyteil 1 bis 3 Kohlenstoffatome enthalten kann.

Die Veresterung oder Amidierung wird zweckmäßigerweise in einem Lösungsmittel wie Methylenchlorid, Chloroform, Ether, Tetrahydrofuran, Dioxan, Toluol oder Dimethylformamid, besonders vorteilhaft jedoch in einem Überschuß einer eingesetzten Verbindung der allgemeinen Formel XI, z.B. in Methanol, Ethanol, n-Propanol, Isopropanol, Ammoniak, Methylamin, Ethylamin, Dimethylamin, Diethylamin oder Piperidin gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxy-succinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25 °C und 150 °C, vorzugsweise jedoch bei Temperaturen zwischen -10 °C und der Siedetemperatur des verwendeten Lösungsmittels, durchgeführt.

Bei den vorstehend beschriebenen Verfahren können vorhandene reaktive Gruppen wie Imino-, Amino-, Alkylamino-, Hydroxy-und/oder Carboxygruppen erforderlichenfalls während der Umsetzung geschützt werden. Als Schutzgruppen kommen beispielsweise für Imino-, Amino- oder Alkylaminogruppen Acetyl-, Benzoyl-, tert.Butoxycarbonyl-, Benzyloxycarbonyl-, Benzyl-, Trityl- oder Fluorenylmethyloxycarbonylgruppen,
für Hydroxygruppen Acetyl-, Benzoyl-, Benzyl-, Trimethylsilyl- oder Tritylgruppen und
für Carboxygruppen Benzyl- oder tert.Butylgruppen in Betracht.

Die gegebenenfalls nach der Umsetzung erforderliche Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser, Dioxan/Wasser oder Eisessig, in Gegenwart einer Säure wie Salzsäure, Schwefelsäure oder Bromwasserstoffsäure oder in Gegenwart einer Alkalibase wie Natriumhydroxid oder Kaliumhydroxid bei Temperaturen zwischen 0 und 150° C, vorzugsweise bei der Siedetemperatur des Reaktionsgemisches. Die Abspaltung eines Benzyl- oder Benzyloxycarbonylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50° C, vorzugsweise jedoch bei Raumtemperatur, und einem Wasserstoffdruck von 1 bis 7 bar, vorzugsweise jedoch von 3 bis 5 bar.

Erhält man erfindungsgemäß eine Verbindung der allgemeinen Formel I, in der $R_4$ eine Alkoxycarbonylgruppe oder eine der eingangs erwähnten Amidocarbonylgruppen darstellt oder enthält, so kann diese mittels Hydrolyse in eine entsprechende Verbindung der allgemeinen Formel I, in der $R_4$ eine Carboxygruppe darstellt oder enthält, übergeführt werden oder eine Verbindung der allgemeinen Formel I, in der $R_4$ eine der eingangs erwähnten Alkoxygruppen darstellt, so kann diese mittels Etherspaltung in eine entsprechende Verbindung der allgemeinen Formel I, in der $R_4$ eine Hydroxygruppe darstellt, übergeführt werden.

Die nachträgliche Hydrolyse wird entweder in Gegenwart einer Säure wie Salzsäure, Schwefelsäure oder Trifluoressigsäure oder in Gegenwart einer Base wie Natriumhydroxid oder Kaliumhydroxid in einem geeigneten Lösungsmittel wie Wasser, Methanol, Ethanol, Ethanol/Wasser, Wasser/Isopropanol oder Wasser/Dioxan bei Temperaturen zwischen -10° C und 120° C, z.B. bei Temperaturen zwischen Raumtemperatur und der Siedetemperatur des Reaktionsgemisches, durchgeführt.

Die nachträgliche Etherspaltung wird in Gegenwart einer Säure wie Chlorwasserstoff, Bromwasserstoff, Schwefelsäure oder Bortribromid in einem geeigneten Lösungsmittel wie Methanol, Ethanol, Wasser/Isopropanol, Methylenchlorid, Chloroform oder Tetrachlorkohlenstoff bei Temperaturen zwischen -30° C und der Siedetemperatur des Reaktionsgemisches oder im Falle eines Benzylethers mit Wasserstoff in Gegenwart eines Hydrierungskatalysators durchgeführt.

Wie bereits eingangs erwähnt, können die neuen Verbindungen in Form ihrer Enantiomeren oder Enantiomerengemische oder, sofern sie mindestens 2 asymmetrische Kohlenstoffatome enthalten, auch in Form ihrer Diastereomeren bzw. Diastereomerengemische vorliegen.

So lassen sich die erhaltenen Verbindungen der allgemeinen Formel I, welche nur ein optisch aktives Zentrum enthalten, nach an sich bekannten Methoden (siehe Allinger N. L. und Elich W. L. in "Topics in Stereochemistry", Vol. 6, Wiley Interscience, 1971) in ihre optischen Antipoden auftrennen, z.B. durch Umkristallisieren aus einem optisch aktiven Lösungsmittel oder durch Umsetzen mit einer, mit der racemischen Verbindung Salze bildenden optisch aktiven Substanz, insbesondere Säuren, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, Di-O-Benzoylweinsäure, Äpfelsäure, Mandelsäure, Camphersulfonsäure, Glutaminsäure, Asparaginsäure oder Chinasäure.

Desweiteren lassen sich die erhaltenen Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen. Ein so erhaltenes Enantiomerenpaar läßt sich anschließend in seine optischen Antipoden, wie oben beschrieben, auftrennen. Enthält beispielsweise eine Verbindung der allgemeinen Formel I zwei optisch aktive Kohlenstoffatome, so erhält man die entsprechenden (R R′, S S′)-und (R S′, S R′)-Formen.

Desweiteren können die erhaltenen Verbindungen der allgemeinen Formel I in ihre Additionssalze, insbesondere für die pharmazeutische Anwendung, in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phorphorsäure, Essigesäure, Oxalsäure, Malonsäure, Fumarsäure, Maleinsäure, Bernsteinsäure, Ascorbinsäure, Äpfelsäure, Weinsäure, Milchsäure, Zitronensäure, Benzoesäure, Methansulfonsäure, Toluolsulfonsäure, Phthalsäure, Naphthalinsulfonsäure, Nikotinsäure, Palmitinsäure oder Embonsäure in Betracht.

Die als Ausgangsstoffe verwendeten Verbindungen der allgemeinen Formeln II bis XI erhält man nach literaturbekannten Verfahren bzw. sind literaturbekannt.

So erhält man beispielsweise eine Ausgangsverbindung der allgemeinen Formel II durch Friedel-Crafts-Acetylierung einer entsprechenden Verbindung, anschließende Bromierung und gegebenenfalls anschließende Umsetzung mit Urotropin und nachfolgende Hydrolyse. Ein so erhaltenes Keton wird anschließend reduziert.

Eine Ausgangsverbindung der allgemeinen Formel IV erhält man durch Umsetzung einer entsprechenden Carbonylverbindung mit einem entsprechenden Amin.

Eine Ausgangsverbindung der allgemeinen Formel V erhält man durch Umsetzung einer entsprechenden Halogenacetyl- oder Glyoxalverbindung mit einem entsprechenden Amin.

Eine Ausgangsverbindung der allgemeinen Formel VIII erhält man durch Umsetzung einer entsprechenden Carbonsäure mit einem entsprechenden Amin in Gegenwart eines die Säure aktivierenden Mittels.

Eine Ausgangsverbindung der allgemeinen Formeln IX und X erhält man durch Umsetzung eines entsprechenden $\alpha$-Halogenalkohols mit einem entsprechenden Amin.

Wie bereits eingangs erwähnt, weisen die neuen Verbindungen der allgemeinen Formel I, deren Enantiomere oder Enantiomerengemische oder, sofern sie mindestens 2 asymmetrische Kohlenstoffatome enthalten, auch deren Diastereomere bzw. Diastereomerengemische und deren Additionssalze, insbesondere für die pharmazeutische Anwendung deren physiologisch verträgliche Säureadditionssalze, wertvolle pharmakologische Eigenschaften auf, insbesondere eine Wirkung auf den Stoffwechsel, vorzugsweise eine blutzuckersenkende, körperfettreduzierende und Energieverbrauch-steigernde Wirkung, sowie eine Senkung der atherogenen Lipoproteine VLDL und LDL.

Beispielsweise wurden die nachfolgenden Verbindungen auf ihre biologischen Eigenschaften wie folgt untersucht:

A = 4′-[2-[N-(2-(3-Chlor-phenyl)-2-hydroxy-ethyl)amino]propyl]biphenylyl-4-carbonsäure-ethylester,

B = 4′-[2-[N-(2-Hydroxy-2-phenyl-ethyl)amino]propyl]biphenylyl-4-oxyessigsäure-methylester,

C = 4′-[2-[N-(2-(3-Chlor-phenyl)-2-hydroxy-ethyl)amino]propyl]biphenylyl-4-oxyessigsäure-methylester,

D = 4′-[2-[N-(2-(3-Chlor-phenyl)-2-hydroxy-ethyl)amino]propyl]-4-(2-hydroxy-ethoxy)biphenyl,

E = 4′-[2-[N-(2-(3-Chlor-phenyl)-2-hydroxy-ethyl)amino]propyl]-4-ethyl-biphenyl,

F = 4′-[2-[N-(2-(3-Chlor-phenyl)-2-hydroxy-ethyl)amino]ethyl]diphenylmethan,

G = 4′-[2-[N-(2-(3-Chlor-phenyl)-2-hydroxy-ethyl)amino]ethyl]diphenylmethan-2-carbonsäure-ethylester und

H = 4′-[2-[N-(2-(3-Chlor-phenyl)-2-hydroxy-ethyl)amino]propyl]diphenylmethan-2-carbonsäure-ethylester.

1. Antidiabetische Wirkung:

Die antidiabetische Wirkung der erfindungsgemäßen Verbindungen läßt sich als eine blutzuckersenkende Wirkung bei Versuchstieren messen. Die zu untersuchenden Substanzen wurden dazu in 1,5%iger Methylzellulose suspendiert und weiblichen Mäusen eigener Zucht mittels Schlundsonde appliziert. 30 Minuten später wurde 1 g Glukose pro kg Körpergewicht in Wasser gelöst und subkutan appliziert. Weitere 30 Minuten später wurde aus dem retroorbitalen Venenplexus Blut entnommen. Aus dem Serum wurde Glukose mit der Hexokinase-Methode mit Hilfe eines Analysenphotometers bestimmt.

In der nachstehenden Tabelle sind die bei dieser Versuchsanordnung beobachteten Blutzuckersenkungen in % einer mitgeführten Kontrollgruppe zusammengestellt. Die statistische Auswertung erfolgte nach dem t-Test nach Student mit $p = 0{,}05$ als Signifikanzgrenze.

| Verbindung | % Änderung vom Wert der Kontrollgruppe | |
|---|---|---|
| | Dosis [mg/kg] | |
| | 1 | 3 |
| A | - 37 | - 49 |
| B | - 29 | - 34 |
| C | - 27 | - 44 |
| D | | - 62 |
| E | - 29 | - 40 |
| F | - 21 | - 39 |
| G | - 35 | - 50 |
| H | | - 62 |

2. Antiadipöse Wirkung:

Die antiadipöse Wirkung der erfindungsgemäßen Verbindungen, die sich in einer Steigerung der Lipolyse ausdrückt, wurde am Anstieg des Glyzerins im Serum gemessen. Der Versuchsablauf ist identisch mit der zur Testung auf blutzuckersenkende Wirkung vorstehend beschriebenen Versuchsanordnung. Glyzerin wurde in einem kombinierten enzymatisch-kolorimetrischen Test mit Hilfe eines Analysenphotometers bestimmt. Die Ergebnisse sind in der nachfolgenden Tabelle in % einer mitgeführten Kontrollgruppe aufgeführt.

| Verbindung | % Änderung vom Wert der Kontrollgruppe | |
|---|---|---|
| | Dosis [mg/kg] | |
| | 1 | 3 |
| A | 121 | 220 |
| B | 194 | 301 |
| C | | 185 |
| D | | 378 |
| E | 141 | 165 |
| F | 80 | 182 |
| G | 143 | 364 |
| H | | 402 |

Desweiteren konnten bei den vorstehend beschriebenen Untersuchungen der erfindungsgemäßen Substanzen bei den applizierten Dosen keine Kreislaufwirkungen und bis zu einer Dosis von 30 mg/kg keine toxischen Nebenwirkungen beobachtet werden. Diese sind daher gut verträglich.

Auf Grund ihrer pharmakologischen Eigenschaften eignen sich daher die neuen Verbindungen der allgemeinen Formel I sowie deren physiologisch verträgliche Additionssalze mit anorganischen oder organischen Säuren zur Behandlung sowohl des Diabetes mellitus als auch der Adipositas, insbesondere also zur Behandlung des adipösen Diabetikers. Hierbei kann die erforderliche Dosis ganz auf den stoffwechselphysiologischen Bedarf des einzelnen Patienten abgestimmt werden, da die Substanzen über einen großen Dosisbereich frei von einer Herz/Kreislaufwirkung sind. Beim Erwachsenen liegen daher die Tagesdosen zwischen 1 und 300 mg, vorzugsweise jedoch bei 1 bis 100 mg, verteilt auf 1 bis 4 Dosen pro Tag. Hierzu lassen sich die obenerwähnten Verbindungen, gegebenenfalls in Kombination mit anderen Wirksubstanzen, in die üblichen galenischen Zubereitungsformen wie Pulver, Tabletten, Dragées, Kapseln, Suppositorien oder Suspensionen einarbeiten.

Die vorstehend erwähnten Verbindungen können ferner als Folge ihrer körperfettreduzierenden

(lipolytischen) Wirkung zur Behandlung fettsüchtiger Tiere und zur Reduktion oder Verhinderung unerwünschten Fettansatzes bei der Mast von Tieren und damit zur Verbesserung der Fleischqualität von Masttieren eingesetzt werden. Weiterhin können die genannten Verbindungen zur Erzielung höherer täglicher Gewichtszunahmen und verbesserter Futterausnutzung in der Tierernährung, vorzugsweise bei der Mast von Tieren, verwendet werden.

Die leistungsfördernde und fettreduzierende Wirkung der vorstehend erwähnten Verbindungen der Formel I wurde beispielshaft an den Substanzen A, C und H wie folgt geprüft:

1.) 4 Gruppen von 20 (Kontrolle) bzw. 10 (Versuchsgruppen A, C und H) männlichen Mäusen, wobei jedes Tier einzeln gehalten wurde, erhielten identisches Futter und Wasser ad libitium. Die Versuchsgruppen erhielten zusätzlich 20 ppm der vorstehend erwähnten Verbindungen (A, C und H) über das Futter verabreicht. Die Versuchsperiode dauerte 14 Tage.

Die nachfolgende Tabelle enthält die gefundenen Ergebnisse als relative Veränderungen gegenüber Kontrolle (Kontrolle = 100):

| | A | C | H |
|---|---|---|---|
| Anfangsgewicht | 101,2 | 100,3 | 99,3 |
| Gewichtszunahme | 90,5 | 183,2 | 239,5 |
| Futteraufnahme | 91,6 | 106,0 | 106,5 |
| Gewichtszunahme/Futteraufnahme | 99,1 | 173,0 | 225,1 |

2.) 4 Gruppen von 20 (Kontrolle) bzw. 10 (Versuchsgruppen A, C und H) männlichen Mäusen, wobei jedes Tier einzeln gehalten wurde, erhielten identisches Futter und Wasser ad libitium. Die Versuchsgruppen erhielten zusätzlich 10 mg/kg Körpergewicht der vorstehend erwähnten Verbindungen p.o.. Die Versuchsperiode dauerte 4 Tage.

Die nachfolgende Tabelle enthält die gefundenen Ergebnisse als relative Veränderungen gegenüber Kontrolle (Kontrolle = 100):

| | A | C | H |
|---|---|---|---|
| Gewichtszunahme/Futteraufnahme | 90,5 | 122,5 | 54,5 |
| Nebenhodenfettdepot | 89,6 | 97,7 | 74,2 |
| Musculus gastrocnemius | 111,4 | 103,3 | 110,7 |
| Musculus soleus | 115,1 | 120,2 | 108,7 |
| Fleisch : Fett-Verhältnis | 126,0 | 112,2 | 147,0 |

Auf Grund dieser Eigenschaften können die vorstehend erwähnten Wirkstoffe als Leistungsförderer bei Tieren zur Förderung und Beschleunigung des Wachstums, der Milch- und Wollproduktion, sowie zur Verbesserung der Futterverwertung, der Schlachtkörperqualität und zur Verschiebung des Fleisch-Fett-Verhältnisses zugunsten von Fleisch eingesetzt werden. Die Wirkstoffe werden bei Nutz-, Zucht-, Zier- und Hobbytieren verwendet.

Zu den Nutz- und Zuchttieren zählen Säugetiere, z.B. Rinder wie Kälber, Ochsen, Bullen, Färsen und Kühe, Büffel, Schweine, Pferde, Schafe, Ziegen, Kaninchen, Hasen, Damwild, Pelztiere wie Nerze und Chinchilla, Geflügel wie Tauben, Hühner, Gänse, Enten, Truthühner, Perlhühner, Fasanen und Wachteln, Fische, wie Karpfen, Forellen, Lachse, Aale, Schleien und Hechte, oder Reptilien wie Schlangen und Krokodile.

Zu den Zier- und Hobbytieren zählen Säugetiere wie Hunde und Katzen, Vögel wie Papageien, Kanarienvögel, Fische wie Zierund Aquarienfische, z.B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichtung des gewünschten Effektes verabreicht wird, kann wegen der günstigen Eigenschaften der Wirkstoffe weitgehend variiert werden. Sie liegt vorzugsweise

bei etwa 0,001 bis 50 mg/kg insbesondere 0,01 bis 10 mg/kg Körpergewicht pro Tag. Die passende Menge des Wirkstoffs sowie die passende Dauer der Verabreichung hängen insbesondere von der Tierart, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Tierart, dem Verhalten und dem Gesundheitszustand der Tiere ab.

Die Wirkstoffe können einmalig verabreicht werden. Die Wirkstoffe können aber auch während der ganzen oder während eines Teils der Wachstumsphase temporär oder kontinuierlich verabreicht werden. Bei kontinuierlicher Verabreichung kann die Anwendung ein- oder mehrmals täglich in regelmäßigen oder unregelmäßigen Abständen erfolgen.

Die Verabreichung erfolgt oral oder parenteral in dafür geeigneten Formulierungen oder in reiner Form. Orale Formulierungen sind Pulver, Tabletten, Granulate, Drenche, Boli sowie Futtermittel, Prämixe für Futtermittel, Formulierungen zur Verabreichung über Trinkwasser.

Die oralen Formulierungen enthalten den Wirkstoff in Konzentrationen von 0,01 ppm - 100 %, bevorzugt von 0,01 ppm - 10 %.

Parenterale Formulierungen sind Injektionen in Form von Lösungen, Emulsionen und Suspensionen, sowie Implantate.

Die Wirkstoffe können in den Formulierungen allein oder in Mischung mit anderen Wirkstoffen, Mineralsalzen, Spurenelementen, Vitaminen, Eiweißstoffen, Farbstoffen, Fetten oder Geschmacksstoffen vorliegen.

Die Konzentration der Wirkstoffe im Futter beträgt normalerweise etwa 0,01 - 500 ppm, bevorzugt 0,1 - 50 ppm.

Die Wirkstoffe können als solche oder in Form von Prämixen oder Futterkonzentraten dem Futter zugesetzt werden.

So enthalten die erfindungsgemäßen Futtermittel neben dem Wirkstoff und gegebenenfalls neben einer üblichen Vitamin-Mineral-Mischung beispielsweise für Mastschweine Gerste, Weizennachmehl, Ackerbohnen, Raps-Extraktionsschrot und Futterfett, für Broiler Mais, Sojabohnenmehl, Fleischmehl, Futterfett und Sojaöl, für Rinder Zuckerrübenschnitzel Maiskleber, Malzkeime, Sojabohnenmehl, Weizen und Molasse sowie für Lämmer Gerste, Sojabohnenmehl, Mais und Melasse. Zu diesem Futter wird eine der eingangs erwähnten Verbindungen der Formel I als Wirkstoff in einer Konzentration von 0,01 bis 500 ppm, vorzugsweise jedoch von 0,1 bis 50 ppm, zugemischt, wobei die Zumischung vorzugsweise in Form einer Wirkstoffvormischung erfolgt. Diese Vormischung enthält beispielsweise pro 10 g 10 mg Wirkstoff zweckmäßigerweise in 9,99 g Maisstärke.

Die nachfolgenden Beispiele sollen die Erfindung näher erläutern:

## Herstellung der Ausgangsverbindungen

### Beispiel A

#### 4′-Acetonyl-biphenylyl-4-carbonsäure-ethylester

a) 4′-(2-Chlor-propionyl)biphenylyl-4-carbonsäure-ethylester

Eine Lösung von 10 g (0,044 Mol) Biphenylyl-4-carbonsäureethylester in 70 ml Methylenchlorid tropft man bei 0° C zu einer Lösung von 25 g (0,187 Mol) Aluminiumchlorid und 9,6 g (0,076 Mol) 2-Chlorpropionylchlorid in 250 ml Methylenchlorid. Nach Stehen über Nacht bei Raumtemperatur gibt man auf Eiswasser und verdünnte Salzsäure und extrahiert mit Methylenchlorid. Die Extrakte werden getrocknet und nach dem Einengen im Fließmittel Cyclohexan/Essigsäure-ethylester (7:1) säulenchromatographisch gereinigt. Dabei werden 6,9 g 4′-(2-Chlor-propionyl)biphenylyl-4-carbonsäure-ethylester erhalten.
Schmelzpunkt: 75-78° C

b) 6,8 g (0,021 Mol) 4′-(2-Chlor-propionyl)biphenylyl-4-carbonsäure-ethylester werden in 40 ml Aceton mit 3,9 g (0,034 Mol) Kaliumacetat 2 Tage am Rückfluß gekocht. Anschließend wird vom Niederschlag abfiltriert, das Filtrat eingeengt und der Einengungsrückstand säulenchromatographisch gereinigt (Fließmittel: Cyclohexan/Essigsäure-ethylester = 7:1). Hierbei werden 5,5 g 4′-(2-Acetoxy-propionyl)-

biphenylyl-4-carbonsäure-ethylester erhalten.

c) 5,5 g (0,016 Mol) 4′-(2-Acetoxy-propionyl)-biphenylyl-4-carbonsäure-ethylester werden in 160 ml Ethanol gelöst und bei 5-10 °C portionsweise mit Natriumborhydrid versetzt (ca. 1 g) bis im Chromatogramm kein Ausgangsmaterial mehr nachweisbar ist. Anschließend wird eingeengt, mit Wasser zer setzt und mit Essigsäure-ethylester extrahiert. Die Extrakte werden eingeengt und nach Zugabe von 50 g Polyphosphorsäure 30 Minuten im Ölbad bei 80 °C gerührt. Danach gibt man auf Eiswasser und extrahiert mit Essigsäure-ethylester. Die Extrakte werden getrocknet, eingeengt und an Kieselgel säulenchromatographisch gereinigt (Fließmittel: Cyclohexan/Essigsäure-ethylester = 3:1).
Ausbeute: 2,8 g (62 % der Theorie)
Schmelzpunkt: 71-73 °C

Analog Beispiel A werden erhalten:
4′-Acetonyl-4-methoxy-biphenyl,
Schmelzpunkt: 89-92 °C
4′-Acetonyl-2-methoxy-biphenyl,
Schmelzpunkt: < 20 °C
4′-Acetonyl-biphenylyl-2-carbonsäure-ethylester,
Schmelzpunkt: < 20 °C
4′-Acetonyl-diphenylmethan-2-carbonsäure-ethylester,
4′-Acetonyl-4-chlor-biphenyl,
Schmelzpunkt: 74-76 °C
4′-Acetonyl-1,2-diphenylethan-2-carbonsäure-ethylester.

Beispiel B

4′-Acetonyl-4-hydroxy-biphenyl

4,8 g (20 mMol) 4′-Acetonyl-4-methoxy-biphenyl werden in 50 ml Benzol gelöst und nach Zugabe von 6,14 g (46 mMol) Aluminiumchlorid 24 Stunden am Rückfluß gekocht. Dann gibt man auf Eis und verdünnte Salzsäure, extrahiert mit Chloroform und reinigt die Extrakte durch Kieselgelchromatographie (Fließmittel: Toluol/Essigsäure-ethylester = 6:1).
Ausbeute: 2,8 g (62 % der Theorie),
Schmelzpunkt: 151-153 °C

Analog Beispiel B wird erhalten:
4′-Acetonyl-2-hydroxy-biphenyl

Beispiel C

4′-Acetonyl-biphenylyl-2-oxyessigsäure-methylester

4,3 g (19 mMol) 4′-Acetonyl-2-hydroxy-biphenyl werden in 50 ml Aceton gelöst und nach Zugabe von 2,63 g (19 mMol) Kaliumcarbonat und 1,8 ml (19 mMol) Bromessigsäure-methylester 5 Stunden am Rückfluß gekocht. Anschließend wird filtriert, das Filtrat eingeengt und der Rückstand an Kieselgel chromatographiert (Fließmittel: Toluol/Essigsäure-ethylester = 10:1).
Ausbeute: 5,0 g (88,2 % der Theorie),
Schmelzpunkt: < 20 °C

Analog Beispiel C wird erhalten:
4′-Acetonyl-biphenylyl-4-oxyessigsäure-methylester,
Schmelzpunkt: 81-82 °C

Beispiel D

4′-(2-Amino-propyl)-4-ethyl-biphenyl

a) 9,0 g (0,0426 Mol) 4-(2-Amino-propyl)biphenyl werden in 50 ml Glykol gelöst und nach Zugabe von 6,3 g (0,0426 Mol) Phthalsäureanhydrid 2 Stunden bei 170° C gerührt. Dann wird mit Wasser verdünnt und das 4-(2-Phthalimido-propyl)biphenyl mit Essigsäure-ethylester extrahiert. Nach dem Einengen der Extrakte und Kristallisation aus Methanol erhält man 10,4 g (72 % der Theorie) vom Schmelzpunkt 143-145° C.

b) 3 g (8,8 mMol) 4-(2-Phthalimido-propyl)biphenyl werden in 50 ml Schwefelkohlenstoff vorgelegt und nach Zugabe von 4 g (0,03 Mol) Aluminiumchlorid tropfenweise mit 0,78 g (0,01 Mol) Acetylchlorid versetzt. Nach Stehenlassen über Nacht gibt man auf Eiswasser/verdünnte Salzsäure und extrahiert mit Chloroform. Durch Einengen der Extrakte und Kristallisation aus Aceton erhält man 1,58 g (47 % der Theorie) 4′-(2-Phthalimido-propyl)-4-acetyl-biphenyl vom Schmelzpunkt 157-159° C.

c) 1,0 g (2,6 mMol) 4′-(2-Phthalimido-propyl)-4-acetyl-biphenyl werden in 25 ml Glykol mit 0,5 ml Hydrazinhydrat und 0,9 g (0,016 Mol) Kaliumhydroxid 3 Stunden bei 200° C gerührt. Dann wird mit Wasser verdünnt, mit Ether extrahiert und die Extrakte eingeengt.
Ausbeute: 0,56 g (90 % der Theorie).

Analog Beispiel D wird erhalten:
4′-(2-Amino-ethyl)-4-ethyl-biphenyl

Durch Hydrazinolyse der entsprechenden Phthalimidoverbindungen werden analog Beispiel Dc erhalten:
4′-(2-Amino-ethyl)-4-methoxy-biphenyl und
4′-(2-Amino-propyl)-4-methoxy-biphenyl

Beispiel E

4′-(2-Amino-propyl)biphenylyl-4-carbonsäure-ethylester

a) 0,9 g (2,3 mMol) 4′-(2-Phthalimido-propyl)-4-acetyl-biphenyl (Beispiel Db) werden in 10 ml Dioxan und 10 ml Wasser suspendiert und nach Zugabe von 0,96 g (24 mMol) gepulvertem Natriumhydroxid unter Kühlung tropfenweise mit 1,43 g (9 mMol) Brom versetzt. Man rührt eine Stunde nach, säuert dann mit Salzsäure an und saugt vom Niederschlag ab. Man erhält 0,8 g (90,3 % der Theorie) an 4′-(2-Phthalimido-propyl)biphenylyl-4-carbonsäure vom Schmelzpunkt 280° C.

b) 2,0 g (5,2 mMol) der vorstehend beschriebenen Verbindung werden in 50 ml konzentrierter Salzsäure suspendiert und im Druckgefäß bei 120° C 6 Stunden geschüttelt. Dann wird zur Trockene eingeengt, mit warmem Aceton digeriert und vom Niederschlag abgesaugt. Der Niederschlag wird in Ethanol gelöst und unter Einleiten von trockenem Salzsäuregas 2 Stunden am Rückfluß gekocht. Anschließend wird eingeengt, alkalisch gestellt und mit Essigsäure-ethylester extrahiert. Nach dem Einengen der Extrakte werden 0,6 g 4′-(2-Amino-propyl)biphenylyl-4-carbonsäure-ethylester erhalten (41 % der Theorie).

Analog Beispiel E wird erhalten:
4′-(2-Amino-ethyl)biphenylyl-4-carbonsäure-ethylester

Beispiel F

4-(2-Amino-pentyl)biphenyl

a) 9,11 g (50 mMol) Biphenylyl-4-aldehyd werden mit 7,4 ml Butylamin in 100 ml Toluol versetzt und 2 Stunden am Wasser abscheider gekocht. Dann wird eingeengt, mit 100 ml Eisessig aufgenommen und nach Zugabe von 10 g (0,096 Mol) Nitrobutan 1 Stunde bei 100° C gerührt. Anschließend wird eingeengt und aus Isopropanol umkristallisiert.
Ausbeute: 85,0 % der Theorie,
Schmelzpunkt: 67-69° C

b) 11,9 g (0,044 Mol) des vorstehend beschriebenen Nitroolefins werden in 50 ml Tetrahydrofuran gelöst und tropfenweise einer siedenden Suspension von 3,8 g (0,1 Mol) Lithiumaluminiumhydrid in 100 ml Tetrahydrofuran zugesetzt. Man rührt eine Stunde nach, zersetzt dann mit 4N Natronlauge, filtriert vom Natriumaluminat ab und engt das Filtrat ein. Die so erhaltene Base wird durch Zugabe von Ethanol/ethanolischer Salzsäure in das Hydrochlorid übergeführt.

Ausbeute: 80 % der Theorie,
Schmelzpunkt: 204-206 °C

Analog Beispiel F, unter Verwendung von Nitromethan bzw. Nitroethan, werden erhalten:
4-(2-Amino-ethyl)biphenyl,
4-(2-Amino-propyl)biphenyl,
4′-(2-Amino-ethyl)-4-methoxy-1,2-diphenylethan,
4′-(2-Amino-propyl)-4-methoxy-1,2-diphenylethan,
4′-(2-Amino-ethyl)-4-methoxy-diphenylmethan,
4′-(2-Amino-ethyl)-4-methoxy-biphenyl.

Beispiel G

4-(2-Amino-ethyl)diphenylmethan

Hergestellt durch Friedel-Crafts-Acylierung von N-Acetyl-2-phenyl-ethylamin mit Benzoylchlorid, nachfolgende Hydrolyse zum 4-(2-Amino-ethyl)benzophenon und anschließende Wolff-Kishner-Reduktion mit Hydrazin (siehe J. Amer. chem. Soc. 76, 5623 (1954)).

Analog Beispiel G werden erhalten:
4′-(2-Amino-propyl)-4-methyl-diphenylmethan,
4-(2-Amino-propyl)-diphenylmethan,
4′-(2-Amino-propyl)-4-chlor-diphenylmethan,
4′-(2-Amino-ethyl)-4-methyl-diphenylmethan,
4′-(2-Amino-ethyl)-4-chlor-diphenylmethan,
4′-(2-Amino-ethyl)diphenylmethan-2-carbonsäure-ethylester (durch nachfolgende Veresterung von 4′-(2-Amino-ethyl)diphenylmethan-2-carbonsäure),
4′-(2-Amino-ethyl)-diphenylmethan-4-carbonsäure-ethylester (durch nachfolgende Veresterung von 4′-(2-Amino-ethyl)diphenylmethan-4-carbonsäure).

Beispiel H

4′-(2-Amino-ethyl)diphenylmethan-2-oxyessigsäure-methylester

a) 15,7 g (0,053 Mol) 4-[2-(Ethoxycarbonylamino)ethyl]benzoesäure-phenylester werden in 80 ml Chlorbenzol gelöst und nach Zugabe von 25 g (0,094 Mol) Aluminiumbromid 5 Stunden bei 110 °C gerührt. Dann gibt man auf Eis/Salzsäure und extrahiert mit Methylenchlorid. Durch Säulenchromatographie an Kieselgel im Fließmittel Cyclohexan/Essigsäure-ethylester = 3:1 wird das 4′-[2-(Ethoxycarbonylamino)-ethyl]-2-hydroxybenzophenon in 46%iger Ausbeute erhalten.

b) 6,5 g (0,021 Mol) der vorstehend beschriebenen Verbindung werden in 50 ml Glykol nach Zusatz von 7,1 g (0,127 Mol) Kaliumhydroxid und 4,1 g Hydrazinhydrat 4 Stunden bei 180 °C gerührt. Dann gibt man auf Eis/Salzsäure, stellt mit Ammoniak alkalisch, extrahiert mit Chloroform und engt ein. Nach Kieselgelchromatographie im Fließmittel Chloroform/Methanol/methanolischer Ammoniak (5:1:0,1) erhält man 4′-(2-Amino-ethyl)-2-hydroxy-diphenylmethan in 26%iger Ausbeute.

c) 11,5 g (0,05 Mol) des vorstehenden Amins werden in 60 ml Dioxan und 60 ml 2N Natronlauge vorgelegt und bei Eiskühlung tropfenweise mit 12 g (0,07 Mol) Chlorameisensäure-benzylester versetzt. Nach Stehen über Nacht wird angesäuert, mit Chloroform extrahiert und nach Einengen der Extrakte an Kieselgel chromatographiert (Fließmittel: Toluol/Essigsäureethylester = 14:1). Man erhält 55 % 4′-[2-(Benzyloxycarbonylamino)ethyl]-2-hydroxy-diphenylmethan.

d) 5 g (0,014 Mol) der vorstehend beschriebenen Verbindung werden in Aceton mit 1,9 g (0,014 Mol) Kaliumkarbonat und 2,1 g (0,014 g) Bromessigsäure-methylester 3 Stunden am Rückfluß gekocht. Dann wird filtriert, eingeengt und mit Chloroform als Fließmittel an Kieselgel chromatographiert. Der dabei isolierte 4′-[2-(Benzyloxycarbonyl-amino)ethyl]diphenylmethan-2-oxyessigsäure-methylester wird anschließend in 40 ml Methanol, nach Zusatz von 8 ml gesättigter methanolischer Salzsäure und 0,4 g Palladium (10%ig auf Kohle), bei Raumtemperatur und einem Wasserstoffdruck von 5 bar hydriert. Nach beendeter Wasserstoffaufnahme wird vom Katalysator abfiltriert und eingeengt. Man erhält 1,9 g 4′-(2-Amino-ethyl)-

diphenylmethan-2-oxyessigsäure-methylester-hydrochlorid.

Analog Beispiel H wird erhalten:

4'-(2-Amino-ethyl)-2-methoxy-diphenylmethan-hydrochlorid

Beispiel 1

4'-[2-[N-(2-(3-Chlor-phenyl)-2-hydroxy-ethyl)amino]propyl]biphenylyl-4-carbonsäure-ethylester

5,6 g (20 mMol) 4'-Acetonyl-biphenylyl-4-carbonsäure-ethylester und 3,4 g (20 mMol) 2-(3-Chlor-phenyl)-2-hydroxy-ethylamin werden in 75 ml Ethanol gelöst und nach Zugabe von 1,25 g (20 mMol) Natriumcyanoborhydrid und 1,2 ml (20 mMol) Eisessig 24 Stunden bei Raumtemperatur gerührt. Anschließend wird eingeengt, auf Wasser gegeben und mit verdünnter Salzsäure angesäuert. Nach 30 Minuten Rühren wird mit Natronlauge alkalisch gestellt und mit Essigsäure-ethylester extrahiert. Die Extrakte werden eingeengt und der Einengungsrückstand an Kieselgel säulenchromatographisch gereinigt (Fließmittel: Essigsäure-ethylester/Methanol = 40:1). Anschließend wird aus Acetonitril umkristallisiert.

Ausbeute: 4,3 g (49 % der Theorie),

Schmelzpunkt: 104-116 °C

| | | | | |
|---|---|---|---|---|
| Ber.: | C 71,30 | H 6,44 | N 3,20 | Cl 8,10 |
| Gef.: | 71,45 | 6,30 | 3,47 | 8,08 |

Laut $^1$H-NMR (400 MHZ) liegt ein 1:1 Diastereomerenpaargemisch vor.

[$CDCl_3$: $\delta$ = 4,56 ppm (dd, 0,5 H), $\delta$ = 4,62 ppm (dd, 0,5 H)]

Analog Beispiel 1 werden folgende Verbindungen hergestellt:

a) 4'-Hydroxy-4-[2-[N-(2-hydroxy-2-phenyl-ethyl)amino]propyl]biphenyl-hydrochlorid

Hergestellt aus 2-Hydroxy-2-phenyl-ethylamin und 4'-Acetonyl-4-hydroxy-biphenyl.

Ausbeute: 50 % der Theorie,

Schmelzpunkt: 154-156 °C

| | | | |
|---|---|---|---|
| Ber.: | C 71,95 | H 6,83 | N 3,65 |
| Gef.: | 71,77 | 6,79 | 3,61 |

Laut $^1$H-NMR (400 MHZ) liegt ein 1:1 Diastereomerenpaargemisch vor.

b) 4'-[2-[N-(2-Hydroxy-2-Phenyl-ethyl)amino]propyl]biphenylyl-4-oxyessigsäure-methylester

Hergestellt aus 2-Hydroxy-2-phenyl-ethylamin und 4'-Acetonylbiphenylyl-4-oxyessigsäure-methylester.

Ausbeute: 23 % der Theorie,

Schmelzpunkt: 90-91 °C

| | | | |
|---|---|---|---|
| Ber.: | C 74,44 | H 6,97 | N 3,34 |
| Gef.: | 74,31 | 7,13 | 3,03 |

Laut $^1$H-NMR (400 MHZ) liegt ein 2:3 Diastereomerenpaargemisch vor. [$CDCl_3$: $\delta$= 4,60 ppm (dd, 0,6 H), $\delta$= 4,66 ppm (dd, 0,4 H)]

c) 4′-[2-[N-(2-(3-Chlor-phenyl)-2-hydroxy-ethyl)amino]propyl]-4-hydroxy-biphenyl

Hergestellt aus 2-(3-Chlor-phenyl)-2-hydroxy-ethylamin und 4′-Acetonyl-4-hydroxy-biphenyl.
Ausbeute: 41 % der Theorie,
Schmelzpunkt: 97-99 °C

| | | | |
|---|---|---|---|
| Ber.: | C 72,34 | H 6,33 | N 3,67 |
| Gef.: | 72,17 | 6,59 | 3,88 |

Laut $^{1}$H-NMR (400 MHZ) liegt ein 5:3 Diastereomerenpaargemisch vor.
[$CDCl_3$: $\delta$ = 4,60 ppm (dd, 0,62 H), $\delta$ = 4,66 ppm (dd, 0,38 H)]

d) 4′-[2-[N-(2-(3-Chlor-phenyl)-2-hydroxy-ethyl)amino]propyl]biphenylyl-4-oxyessigsäure-methylester

Hergestellt aus 2-(3-Chlor-phenyl)-2-hydroxy-ethyl)amin und 4′-Acetonyl-biphenylyl-4-oxyessigsäure-methylester.
Ausbeute: 25 % der Theorie,
Schmelzpunkt: 106-109 °C

| | | | | |
|---|---|---|---|---|
| Ber.: | C 68,79 | H 6,22 | N 3,09 | Cl 7,81 |
| Gef.: | 68,90 | 6,06 | 3,07 | 7,62 |

Laut $^{1}$H-NMR (400 MHZ) liegt ein 1:1 Diastereomerenpaargemisch vor.
[$CDCl_3$: $\delta$ = 4,55 ppm (dd), $\delta$ = 4,615 ppm (dd)]

e) 4′-[2-[N-(2-(3-Chlor-phenyl)-2-hydroxy-ethyl)amino]propyl]-4-methoxy-biphenyl

Hergestellt aus 2-(3-Chlor-phenyl)-2-hydroxy-ethylamin und 4′-Acetonyl-4-methoxy-biphenyl.
Ausbeute: 20,1 % der Theorie,
Schmelzpunkt: 109-111 °C

| | | | | |
|---|---|---|---|---|
| Ber.: | C 72,81 | H 6,62 | N 3,54 | Cl 8,95 |
| Gef.: | 72,80 | 6,64 | 3,57 | 8,82 |

Laut $^{1}$H-NMR (400 MHZ) liegt eir 1:3 Diastereomerenpaargemisch vor.
[$CDCl_3$: $\delta$ = 4,55 ppm (dd, 0,75 H), $\delta$ = 4,62 ppm (dd, 0,25 H)]

f) 4′-Chlor-4-[2-[N-(2-(3-Chlor-phenyl)-2-hydroxy-ethyl)amino]propyl]biphenyl

Hergestellt aus 4′-Acetonyl-4-chlor-biphenyl und 2-(3-Chlorphenyl)-2-hydroxy-ethylamin.
Ausbeute: 26 % der Theorie,
Schmelzpunkt: 115-117 °C

| | | | | |
|---|---|---|---|---|
| Ber.: | C 69,00 | H 5,79 | N 3,50 | Cl 17,71 |
| Gef.: | 69,10 | 5,74 | 3,34 | 17,98 |

Laut $^1$H-NMR (400 MHZ) liegt ein 3:4 Diastereomerenpaargemisch vor. [$CDCl_3$: $\delta$ = 4,56 ppm (dd, 0,57 H), $\delta$ = 4,62 ppm (dd, 0,43 H)]

g) 4′-[2-[N-(2-Hydroxy-2-phenyl-ethyl)amino]propyl]biphenylyl-4-carbonsäure-ethylester

Hergestellt aus 2-Hydroxy-2-phenyl-ethylamin und 4′-Acetonylbiphenylyl-4-carbonsäure-ethylester.
Ausbeute: 62,5 % der Theorie,
Schmelzpunkt: 96-98 ° C

| | | | |
|---|---|---|---|
| Ber.: | C 77,39 | H 7,24 | N 3,47 |
| Gef.: | 77,20 | 7,08 | 3,29 |

Laut $^1$H-NMR (400 MHZ) liegt ein 1:1 Diastereomerenpaargemisch vor. [$CDCl_3$: $\delta$ = 4,62 ppm (dd, 0,5 H), $\delta$ = 4,67 ppm (dd, 0,5 H)]

h) 4′-[2-[N-(2-(3-Chlor-phenyl)-2-hydroxy-ethyl)amino]propyl]-biphenylyl-2-carbonsäure-ethylester

Hergestellt aus 2-(3-Chlor-pheryl)-2-hydroxy-ethylamin und 4′-Acetonyl-biphenylyl-2-carbonsäure-ethylester.
Ausbeute: 39,3 % der Theorie,
Schmelzpunkt: < 20 ° C

| | | | | | | |
|---|---|---|---|---|---|---|
| Ber.: | C | 71,30 | H | 6,44 | N | 3,20 |
| Gef.: | | 71,55 | | 6,27 | | 3,09 |

Laut $^1$H-NMR (400 MHZ) liegt ein 1:1 Diastereomerenpaargemisch vor. [$CDCl_3$: $\delta$ = 4,50 ppm (dd, 0,5 H), $\delta$ = 4,60 ppm (dd, 0,5 H)]

i) 4′-[2-[N-(2-(3-Chlor-phenyl)-2-hydroxy-ethyl)amino]propyl]-2-methoxy-biphenyl

Hergestellt aus 2-(3-Chlor-phenyl)-2-hydroxy-ethylamin und 4′-Acetonyl-2-methoxy-biphenyl.
Ausbeute: 88,4 % der Theorie,
Schmelzpunkt: < 20 ° C

| | | | | | | |
|---|---|---|---|---|---|---|
| Ber.: | C | 72,81 | H | 6,62 | N | 3,54 |
| Gef.: | | 72,61 | | 6,60 | | 3,62 |

Laut $^1$H-NMR (400 MHZ) liegt ein 1:1 Diastereomerenpaargemisch vor. [$CDCl_3$: $\delta$ = 4,61 ppm (dd, 0,5 H), $\delta$ = 4,55 ppm (dd, 0,5 H)]

k) 4′-[2-[N-(2-(3-Chlor-phenyl)-2-hydroxy-ethyl)amino]propyl]-biphenylyl-2-oxyessigsäure-methylester

Hergestellt aus 2-(3-Chlor-phenyl)-2-hydroxy-ethylamin und 4′-Acetonyl-biphenylyl-2-oxyessigsäure-methylester.
Ausbeute: 83 % der Theorie,
Schmelzpunkt: < 20 ° C

| | | | | | | |
|---|---|---|---|---|---|---|
| Ber.: | C | 68,79 | H | 6,22 | N | 3,08 |
| Gef.: | | 68,80 | | 6,10 | | 2,91 |

Laut $^1$H-NMR (400 MHZ) liegt ein 1:1 Diastereomerenpaargemisch vor.
[$CDCl_3$: $\delta$ = 4,686 ppm (dd, 0,5 H), $\delta$ = 4,62 ppm (dd, 0,5 H)]

l) 4'-[2-[N-(2-(3-Chlor-phenyl)-2-hydroxy-ethyl)amino]propyl]-1,2-diphenylethan-2-carborsäure-ethylester

Hergestellt aus 2-(3-Chlor-phenyl)-2-hydroxy-ethylamin und 4'-Acetonyl-1,2-diphenylethan-2-carbonsäure-ethylester.
Ausbeute: 67 % der Theorie,
Schmelzpunkt: < 20 °C

| | | | | |
|---|---|---|---|---|
| Ber.: | C 72,16 | H 7,12 | N 3,01 | Cl 7,61 |
| Gef.: | 72,40 | 7,18 | 2,93 | 7,41 |

Laut $^1$H-NMR (400 MHZ) liegt ein 1:1 Diastereomerenpaargemisch vor.
[$CDCl_3$: $\delta$= 4,54 ppm (dd, 0,5 H), $\delta$ = 4,62 ppm (dd, 0,5 H)]

m) 4'-[2-[N-(2-(3-Chlor-phenyl)-2-hydroxy-ethyl)amino]propyl]diphenylmethan-2-carbonsäure-ethylester

Hergestellt aus 2-(3-Chlor-phenyl)-2-hydroxy-ethylamin und 4'-Acetonyl-diphenylmethan-2-carbonsäure-ethylester. Ausbeute: 83 % der Theorie,
Schmelzpunkt: < 20 °C

| | | | | |
|---|---|---|---|---|
| Ber.: | C 71,75 | H 6,69 | N 3,10 | Cl 7,84 |
| Gef.: | 71,45 | 6,87 | 2,94 | 8,03 |

Laut $^1$H-NMR (400 MHZ) liegt ein 1:1 Diastereomerenpaargemisch vor.
[$CDCl_3$: $\delta$ = 4,51 ppm (dd, 0,5 H), $\delta$= 4,59 ppm (dd, 0,5 H)]

Beispiel 2

4'-[2-[N-(2-Hydroxy-2-phenyl-ethyl)amino]ethyl]-4-methoxy-biphenyl

4 g (20 mMol) Phenacylbromid werden in 20 ml Methylenchlorid gelöst und tropfenweise zu einer Lösung von 4,6 g (20 mMol) 4'-(2-Amino-ethyl)-4-methoxy-biphenyl und 3,45 ml (20 mMol) N,N-Diisopropyl-ethylamin in lOC ml Methylenchlorid bei Raumtemperatur gegeben. Nach 2 Stunden werden 50 ml Methanol zugesetzt und unter leichter Kühlung portionsweise 1 g (27 mMol) Natriumborhydrid eingetragen. Nach Rühren über Nacht wird im Vakuum eingeengt, mit verdünnter Salzsäure angesäuert und 30 Minuten gerührt. Anschließend stellt man mit Natronlauge alkalisch und extrahiert mit Chloroform. Der Chloroform-Einengungsrückstand wird an Kieselgel säulenchromatographisch gereinigt (Fließmittel: Chloroform/Methanol = 10:1). Zuletzt wird aus Acetonitril umkristallisiert.
Ausbeute: 1,1 g (16 % der Theorie),
Schmelzpunkt: 137-138 °C

| | | | |
|---|---|---|---|
| Ber.: | C 79,51 | H 7,25 | N 4,03 |
| Gef.: | 79,62 | 7,19 | 3,97 |

Analog Beispiel 2 werden folgende Verbindungen hergestellt:

a) 4′-[2-[N-(2-(3-Chlor-phenyl)-2-hydroxy-ethyl)amino]ethyl]-4-hydroxy-biphenyl-semihydrat

Hergestellt aus 3-Chlor-phenacylbromid und 4′-(2-Aminoethyl)-4-hydroxy-biphenyl.
Ausbeute: 13 % der Theorie,
Schmelzpunkt: 114-117 °C

| | | | |
|---|---|---|---|
| Ber.: | C 70,09 | H 6,15 | N 3,72 |
| Gef.: | 69,90 | 6,11 | 3,74 |

b) 4′-[2-[N-(2-(3-Chlor-phenyl)-2-hydroxy-ethyl)amino]ethyl]-4-methoxy-biphenyl

Hergestellt aus 3-Chlor-phenacylbromid und 4′-(2-Aminoethyl)-4-methoxy-biphenyl.
Ausbeute: 34 % der Theorie,
Schmelzpunkt: 114-116 °C

| | | | | |
|---|---|---|---|---|
| Ber.: | C 72,34 | H 6,33 | N 3,67 | Cl 9,28 |
| Gef.: | 72,40 | 6,17 | 3,53 | 9,08 |

c) 4′-[2-[N-(2-(3-Chlor-phenyl)-2-hydroxy-ethyl)amino]ethyl]biphenylyl-4-carbonsäure-ethylester

aus 3-Chlor-phenacylbromid und 4′-(2-Amino-ethyl)biphenylyl-4-carbonsäure-ethylester.
Ausbeute: 14 % der Theorie,
Schmelzpunkt: 121-122 °C

| | | | | |
|---|---|---|---|---|
| Ber.: | C 70,83 | H 6,18 | N 3,30 | Cl 8,36 |
| Gef.: | 70,90 | 6,19 | 3,35 | 8,39 |

d) 4-[2-[N-(2-(3-Chlor-phenyl)-2-hydroxy-ethyl)amino]propyl]biphenyl

Hergestellt aus 3-Chlor-phenacylbromid und 4-(2-Amino-propyl)biphenyl.
Ausbeute: 23 % der Theorie,
Schmelzpunkt: 95-96 °C

| | | | |
|---|---|---|---|
| Ber.: | C 75,50 | H 6,61 | N 3,89 |
| Gef.: | 75,70 | 6,69 | 3,93 |

Laut $^1H$-NMR (400 MHZ) liegt ein 1:2 Diastereomerenpaargemisch vor. [$CDCl_3$: $\delta$ = 4,57 ppm (dd, 0,66 H), $\delta$ = 4,63 ppm (dd, 0,33 H)]

e) 4′-[2-[N-(2-(3-Chlor-phenyl)-2-hydroxy-ethyl)amino]propyl]-4-ethyl-biphenyl

Hergestellt aus 4′-(2-Amino-propyl)-4-ethyl-biphenyl und 3-Chlor-phenacylbromid.
Ausbeute: 26 % der Theorie,
Schmelzpunkt: 104-114° C

| | | | | |
|---|---|---|---|---|
| Ber.: | C 76,22 | H 7,16 | N 3,56 | Cl 9,00 |
| Gef.: | 76,37 | 7,30 | 3,53 | 9,12 |

Laut $^1H$-NMR (400 MHZ) liegt ein 2:1 Diastereomerenpaargemisch vor. [$CDCl_3$: $\delta$ = 4,56 ppm (dd, 0,33 H), $\delta$ = 4,62 ppm (dd, 0,66 H)]

f) 4′-[2-[N-(2-(3-Chlor-phenyl)-2-hydroxy-ethyl)amino]propyl]biphenylyl-4-carbonsäure-ethylester

Hergestellt aus 4′-(2-Amino-propyl)biphenylyl-4-carbonsäureethylester und 3-Chlor-phenacylbromid.
Ausbeute: 25 % der Theorie,
Schmelzpunkt: 94-96° C

| | | | |
|---|---|---|---|
| Ber.: | C 71,30 | H 6,44 | N 3,20 |
| Gef.: | 71,45 | 6,52 | 3,37 |

Laut $^1H$-NMR (400 MHZ) liegt ein 1:2 Diastereomerenpaargemisch vor. [$CDCl_3$: $\delta$ = 4,56 ppm (dd, 0,33 H), $\delta$ = 4,62 ppm (dd, 0,66 H)]

g) 4-[2-[N-(2-(3-Chlor-phenyl)-2-hydroxy-ethyl)amino]ethyl]biphenyl

Hergestellt aus 3-Chlor-phenacylbromid und 4-(2-Amino-ethyl)biphenyl.
Ausbeute: 23 % der Theorie,
Schmelzpunkt: 109-110° C

| | | | | |
|---|---|---|---|---|
| Ber.: | C 75,10 | H 6,30 | N 3,98 | Cl 10,07 |
| Gef.: | 74,99 | 6,30 | 3,97 | 10,28 |

h) 4′-[2-[N-(2-(3-Chlor-phenyl)-2-hydroxy-ethyl)amino]ethyl]-4-ethyl-biphenyl

Hergestellt aus 3-Chlor-phenacylbromid und 4′-(2-Aminoethyl)-4-ethyl-biphenyl.
Ausbeute: 23 % der Theorie,
Schmelzpunkt: 123-125° C

| | | | | |
|---|---|---|---|---|
| Ber.: | C 75,97 | H 6,90 | N 3,69 | Cl 9,33 |
| Gef.: | 75,95 | 6,78 | 3,58 | 9,37 |

i) 4-[2-[N-(2-(3-Chlor-phenyl)-2-hydroxy-ethyl)amino]ethyl]diphenylmethan

Hergestellt aus 4-(2-Amino-ethyl)diphenylmethan und 3-Chlorphenacylbromid.
Ausbeute: 49 % der Theorie,
Schmelzpunkt: 86-87 °C

| | | | | |
|---|---|---|---|---|
| Ber.: | C 75,50 | H 6,61 | N 3,83 | Cl 9,69 |
| Gef.: | 75,74 | 6,41 | 3,96 | 9,53 |

j) 4′-[2-[N-(2-(3-Chlor-phenyl)-2-hydroxy-ethyl)amino]ethyl]-4-methoxy-diphenylmethan

Hergestellt aus 3-Chlor-phenacylbromid und 4′-(2-Aminoethyl)-4-methoxy-diphenylmethan.
Ausbeute: 22,5 % der Theorie,
Schmelzpunkt: 90-92 °C

| | | | | |
|---|---|---|---|---|
| Ber.: | C 72,81 | H 6,62 | N 3,54 | Cl 8,95 |
| Gef.: | 72,66 | 6,51 | 3,53 | 8,93 |

k) 4′-[2-[N-(2-(3-Chlor-phenyl)-2-hydroxy-ethyl)amino]ethyl]-4-methyl-diphenylmethan

Hergestellt aus 3-Chlor-phenacylbromid und 4′ (2-Aminoethyl)-4-methyl-diphenylmethan.
Ausbeute: 25,5 % der Theorie,
Schmelzpunkt: 100-102 °C

| | | | | |
|---|---|---|---|---|
| Ber.: | C 75,87 | H 6,90 | N 3,69 | Cl 9,33 |
| Gef.: | 75,56 | 6,94 | 3,77 | 9,43 |

l) 4′-[2-[N-(2-(3-Chlor-phenyl)-2-hydroxy-ethyl)amino]ethyl]diphenylmethan-4-carbonsäure-ethylester

Hergestellt aus 3-Chlor-phenacylbromid und 4′-(2-Aminoethyl)-diphenylmethan-4-carbonsäure-ethylester.
Ausbeute: 19 % der Theorie,
Schmelzpunkt: 97-98 °C

| | | | | |
|---|---|---|---|---|
| Ber.: | C 70,97 | H 6,39 | N 3;20 | Cl 8.09 |
| Gef.: | 71,14 | 6,44 | 3,29 | 8,14 |

m) 4′-Chlor-4-[2-[N-(2-(3-chlor-phenyl)-2-hydroxy-ethyl)amino]ethyl]diphenylmethan

Hergestellt aus 4′-(2-Amino-ethyl)-4-chlor-diphenylmethan und 3-Chlor-phenacylbromid.
Ausbeute: 16 % der Theorie,
Schmelzpunkt: 86-88° C

| | | | | |
|---|---|---|---|---|
| Ber.: | C 69,01 | H 5,79 | N 3,50 | Cl 17,71 |
| Gef.: | 69,11 | 5,72 | 3,45 | 17,66 |

n) 4-[2-[N-(2-Hydroxy-2-phenyl-ethyl)amino]propyl]diphenylmethan

Hergestellt aus 4-(2-Amino-propyl)diphenylmethan und Phenacylbromid.
Ausbeute: 17 % der Theorie,
Schmelzpunkt: 86-88° C

| | | | |
|---|---|---|---|
| Ber.: | C 83,44 | H 7,88 | N 4,05 |
| Gef.: | 83,35 | 8,03 | 4,06 |

Laut $^1$H-NMR (400 MHZ) liegt ein 45:55 Diastereomerenpaargemisch vor.
[$CDCl_3$: $\delta$ = 4,57 ppm (dd, 0,45 H), $\delta$ = 4,64 ppm (dd, 0,55 H)]

o) 4-[2-[N-(2-(3-Chlor-phenyl)-2-hydroxy-ethyl)amino]propyl]diphenylmethan

Hergestellt aus 4-(2-Amino-propyl)diphenylmethan und 3-Chlorphenacylbromid.
Ausbeute: 17,9 % der Theorie,
Schmelzpunkt: 92-96° C

| | | | | |
|---|---|---|---|---|
| Ber.: | C 75,87 | H 6,80 | N 3,69 | Cl 9,33 |
| Gef.: | 75,70 | 6,97 | 3,57 | 9,46 |

Laut $^1$H-NMR (400 MHZ) liegt ein 1:1 Diastereomerenpaargemisch vor.
[$CDCl_3$: $\delta$ = 4,52 ppm (dd), $\delta$ = 4,58 ppm (dd)]

p) 4′-Chlor-4-[2-[N-(2-(3-chlor-phenyl)-2-hydroxy-ethyl)amino]propyl]diphenylmethan

Hergestellt aus 3-Chlor-phenacylbromid und 4′-(2-Amino-propyl)-4-chlor-diphenylmethan.
Ausbeute: 19,2 % der Theorie,
Schmelzpunkt: 85-90° C

| | | | | |
|---|---|---|---|---|
| Ber.: | C 69,57 | H 6,08 | N 3,38 | Cl 17,11 |
| Gef.: | 69,70 | 6,01 | 3,26 | 17,24 |

Laut ¹H-NMR (400 MHZ) liegt ein 20:1 Diastereomerenpaargemisch vor.
[$CDCl_3$: $\delta$ = 4,54 ppm (dd, 0,05 H), $\delta$ = 4,61 ppm (dd, 0,95 H)]

q) 4'-[2-[N-(2-Hydroxy-2-phenyl-ethyl)amino]ethyl]benzhydrol-4'-carbonsäure-äthylester

Hergestellt aus Phenacylbromid und 4'-(2-Amino-ethyl)benzophenon-4-carbonsäure-ethylester.
Ausbeute: 14,6 % der Theorie,
Schmelzpunkt: 111-113 °C

| | | | |
|---|---|---|---|
| Ber.: | C 74,80 | H 6,52 | N 3,35 |
| Gef.: | 75,00 | 6,65 | 3,49 |

r) 4-[2-[N-(2-(3-Chlor-phenyl)-2-hydroxy-ethyl)amino]-2-methyl-propyl]biphenyl

Hergestellt aus 3-Chlor-phenacylbromid und 4-(2-Amino-2-methyl-propyl)biphenyl.
Ausbeute: 13 % der Theorie,
Schmelzpunkt: 80 °C

| | | | | |
|---|---|---|---|---|
| Ber.: | C 75,87 | H 6,90 | N 3,69 | Cl 9,33 |
| Gef.: | 76,00 | 6,96 | 3,68 | 9,25 |

s) 4-[2-[N-(2-(3-Chlor-phenyl)-2-hydroxy-ethyl)amino]pentyl]biphenyl-hydrochlorid

Hergestellt aus 3-Chlor-phenacylbromid und 4-(2-Aminopentyl)biphenyl-hydrochlorid.
Ausbeute: 11,5 % der Theorie,
Schmelzpunkt: 164-167 °C

| | | | | |
|---|---|---|---|---|
| Ber.: | C 69,77 | H 6,79 | N 3,25 | Cl 16,48 |
| Gef.: | 69,74 | 6,74 | 3,21 | 16,45 |

Laut ¹H-NMR (400 MHZ) liegt ein 2:1 Diastereomerenpaargemisch vor.
[$CDCl_3/CD_3OD$: $\delta$ = 5,10 ppm (dd, 0,66 H), $\delta$ = 5,17 ppm (dd, 0,33 H)]

t) 4'-[2-[N-(2-(4-Amino-3-cyano-5-fluor-phenyl)-2-hydroxy-ethyl)amino]ethyl]-4-methoxy-biphenyl

Hergestellt aus 4 -Amino-3-cyano-5-fluor-phenacylbromid und 4'-(2-Amino-ethyl)-4-methoxy-biphenyl.
Ausbeute: 22,3 % der Theorie,
Schmelzpunkt: 150-153 °C

| | | | |
|---|---|---|---|
| Ber.: | C 71,09 | H 5,97 | N 10,36 |
| Gef.: | 70,93 | 5,91 | 10,24 |

u) 4′-[2-[N-(2-(4-Amino-3-cyano-5-fluor-phenyl)-2-hydroxy-ethyl)amino]ethyl]biphenylyl-4-carbonsäure-ethylester

Hergestellt aus 4-Amino-3-cyano-5-fluor-phenacylbromid und 4′-(2-Amino-ethyl)biphenylyl-4-carbonsäure-ethylester.
Ausbeute: 18 % der Theorie,
Schmelzpunkt: 153-154 °C

| | | | |
|---|---|---|---|
| Ber.: | C 69,78 | H 5,86 | N 9,39 |
| Gef.: | 69,59 | 5,64 | 9,23 |

v) 4′-[2-[N-(2-(4-Amino-3,5-dichlor-phenyl)-2-hydroxy-ethyl)amino]ethyl]biphenylyl-4-carbonsäure-ethylester

Hergestellt aus 4-Amino-3,5-dichlor-phenacylbromid und 4′-(2-Amino-ethyl)biphenylyl-4-carbonsäure-ethylester.
Ausbeute: 17,6 % der Theorie,
Schmelzpunkt: 123-125 °C

| | | | | |
|---|---|---|---|---|
| Ber.: | C 63,70 | H 5,13 | N 5,94 | Cl 15,04 |
| Gef.: | 63,78 | 5,24 | 5,95 | 15,22 |

w) 4′-[2-[N-(2-(4-Amino-3,5-dichlor-phenyl)-2-hydroxy-ethyl)amino]ethyl]-4-methoxy-biphenyl

Hergestellt aus 4-Amino-3,5-dichlor-phenacylbromid und 4′-(2-Amino-ethyl)-4-methoxy-biphenyl.
Ausbeute: 17 % der Theorie,
Schmelzpunkt: 150-151 °C

| | | | | |
|---|---|---|---|---|
| Ber.: | C 64,04 | H 5,61 | N 6,49 | Cl 16,44 |
| Gef.: | 64,17 | 5,78 | 6,45 | 16,44 |

x) 4′-[2-[N-(2-(4-Amino-3,5-dichlor-phenyl)-2-hydroxy-ethyl)amino]ethyl]-4-hydroxy-biphenyl

Hergestellt aus 4-Amino-3,5-dichlor-phenacylbromid und 4′-(2-Amino-ethyl)-4-hydroxy-biphenyl.
Ausbeute: 11 % der Theorie,
Schmelzpunkt: 88-90 °C

| | | | | |
|---|---|---|---|---|
| Ber.: | C 63,32 | H 5,31 | N 6,71 | Cl 16,99 |
| Gef.: | 63,35 | 5,58 | 6,81 | 16,63 |

y) 4′-[2-[N-(2-(3-Chlor-phenyl)-2-hydroxy-ethyl)amino]ethyl]diphenylmethan-2-carbonsäure-ethylester

Hergestellt aus 3-Chlor-phenacylbromid und 4′-(2-Aminoethyl)-diphenylmethan-2-carbonsäure-ethylester.
Ausbeute: 30,4 % der Theorie,
Schmelzpunkt: < 20 °C

| | | | |
|---|---|---|---|
| Ber.: | C 71,30 | H 6,44 | N 3,20 |
| Gef.: | 71,36 | 6,44 | 3,37 |

z) 4′-[2-[N-(2-(3-Chlor-phenyl)-2-hydroxy-ethyl)amino]propyl]-4-methoxy-1,2-diphenylethan

Hergestellt aus 3-Chlor-phenacylbromid und 4′-(2-Amino-propyl)-4-methoxy-1,2-diphenylethan.
Ausbeute: 17 % der Theorie,
Schmelzpunkt: 108-110 °C

| | | | | |
|---|---|---|---|---|
| Ber.: | C 73,65 | H 7,13 | N 3,30 | Cl 8,36 |
| Gef.: | 73,45 | 7,19 | 3,50 | 8,40 |

Laut $^1$H-NMR (400 MHZ) liegt ein 4:1 Diastereomerenpaargemisch vor.
[$CDCl_3/CD_3OD$: $\delta$ = 4,59 ppm (dd, 0,8 H), $\delta$ = 4,52 ppm (dd, 0,2 H)]

aa) 4′-[2-[N-(2-(3-Chlor-phenyl)-2-hydroxy-ethyl)amino]ethyl]-4-methoxy-1,2-diphenylethan

Hergestellt aus 3-Chlor-phenacylbromid und 4′-(2-Aminoethyl)-4-methoxy-1,2-diphenylethan.
Ausbeute: 27,8 % der Theorie,
Schmelzpunkt: 112-113 °C

| | | | | |
|---|---|---|---|---|
| Ber.: | C 73,24 | H 6,88 | N 3,42 | Cl 8,65 |
| Gef.: | 73,45 | 6,92 | 3,28 | 8,60 |

ab) 4′-[2-[N-(2-(3-Chlor-phenyl)-2-hydroxy-ethyl)amino]ethyl]diphenylmethan-2-oxyessigsäure-methylester

Hergestellt aus 3-Chlor-phenacylbromid und 4′-(2-Aminoethyl)-diphenylmethan-2-oxyessigsäure-ethylester-hydrochlorid unter Zusatz von N,N-Diisopropyl-ethylamin.
Ausbeute: 14 % der Theorie,
Schmelzpunkt: 84-86 °C

| | | | | |
|---|---|---|---|---|
| Ber.: | C 68,80 | H 6,22 | N 3,09 | Cl 7,81 |
| Gef.: | 68,61 | 6,05 | 3,07 | 8,01 |

ac) 4′-[2-[N-(2-(3-Chlor-phenyl)-2-hydroxy-ethyl)amino]ethyl]-2-methoxy-diphenylmethan

Hergestellt aus 3-Chlor-phenacylbromid und 4′-(2-Aminoethyl)-2-methoxy-diphenylmethan-hydrochlorid

unter Zusatz von N,N-Diisopropyl-ethylamin.
Ausbeute: 16 % der Theorie,
Schmelzpunkt: 73-75 °C

| | | | |
|---|---|---|---|
| Ber.: | C 72,80 | H 6,62 | N 3,54 |
| Gef.: | 72,53 | 6,61 | 3,44 |

Beispiel 3

4′-[2-[N-(2-Hydroxy-2-phenyl-ethyl)amino]ethyl]biphenylyl-4-carbonsäure-ethylester

1,0 g (3,27 mMol) 4′-(2-Amino-ethyl)biphenylyl-4-carbonsäure-ethylester-hydrochlorid werden in 10 ml absolutem Ethanol mit 367 mg (3,27 mMol) Kalium-tert.butylat versetzt und unter Stickstoff auf Rückflußtemperatur erhitzt. Nach 5 Minuten tropft man 0,37 ml (3,27 mMol) Styroloxid, gelöst in 10 ml absolutem Ethanol, zu. Anschließend erhitzt man weitere 3 Stunden am Rückfluß. Nach dem Abkühlen wird eingeengt und im Fließmittel Chloroform/Methanol = 15:1 an Kieselgel säulenchromatographisch gereinigt. Zum Schluß wird aus Acetonitril umkristallisiert.
Ausbeute: 270 mg (21,3 % der Theorie),
Schmelzpunkt: 116-117 °C

| | | | |
|---|---|---|---|
| Ber.: | C 77,09 | H 6,99 | N 3,60 |
| Gef.: | 77,10 | 6,92 | 3,57 |

Analog Beispiel 3 werden folgende Verbindungen hergestellt:

a) 4′-Hydroxy-4-[2-[N-(2-hydroxy-2-phenyl-ethyl)amino]ethyl]biphenyl

Hergestellt aus Styroloxid und 4′-(2-Amino-ethyl)-4-hydroxybiphenyl.
Ausbeute: 14,2 % der Theorie,
Schmelzpunkt: 134-136 °C

| | | | |
|---|---|---|---|
| Ber.: | C 79,25 | H 6,95 | N 4,20 |
| Gef.: | 79,10 | 7,06 | 4,24 |

b) 4-[2-[N-(2-Hydroxy-2-phenyl-ethyl)amino]propyl]biphenyl

Hergestellt aus Styroloxid und 4-(2-Amino-propyl)biphenyl.
Ausbeute: 27 % der Theorie,
Schmelzpunkt: 96-100 °C

| | | | |
|---|---|---|---|
| Ber.: | C 83,34 | H 7,60 | N 4,23 |
| Gef.: | 83,30 | 7,52 | 4,22 |

Laut $^{1}H$-NMR (400 MHZ) liegt ein 1:6 Diastereomerenpaargemisch vor.
[$CDCl_3$: $\delta$ = 4,61 ppm (dd, 0,85 H), $\delta$= 4,67 ppm (dd, 0,15 H)]

c) 4-[2-[N-(2-Hydroxy-2-phenyl-ethyl)amino]ethyl]benzophenon-hydrochlorid

Hergestellt aus Styroloxid und 4-(2-Amino-ethyl)benzophenon-hydrochlorid.
Ausbeute: 17 % der Theorie,
Schmelzpunkt: 169-172° C

| | | | | |
|---|---|---|---|---|
| Ber.: | C 72,34 | H 6,33 | N 3,67 | Cl 9,28 |
| Gef.: | 72,50 | 6,13 | 3,78 | 9,57 |

Beispiel 4

4'-[2-[N-(2-Hydroxy-2-phenyl-ethyl)amino]ethyl]-4-methoxy-diphenylmethan

2,82 g (11 mMol) 4-[4-Methoxy-benzyl]phenylessigsäure werden in 25 ml Chloroform gelöst und nach Zugabe von 2,5 ml Thionylchlorid 30 Minuten bei 50° C gerührt. Dann wird eingeengt, in 30 ml Chloroform aufgenommen und unter Kühlen, zu einer Lösung von 1,37 g (10 mMol) 2-Hydroxy-2-phenyl-ethylamin und 2,1 ml (15 mMol) Triethylamin in 40 ml Chloroform getropft. Nach 2 Stunden Rühren bei Raumtemperatur wird zuerst mit verdünnter Natronlauge dann mit verdünnter Salzsäure gewaschen. Anschließend werden die Chloroformextrakte getrocknet und eingeengt. Der Einengungsrückstand, bestehend aus N-(2-Hydroxy-2-phenyl-ethyl)-4-(4-methoxy-benzyl)phenylacetamid, wird in 20 ml Tetrahydrofuran gelöst und unter Stickstoff zu 1,33 g (35 mMol) Lithiumaluminiumhydrid in 20 ml Tetrahydrofuran getropft. Nach 4 Stunden Erhitzen am Rückfluß wird mit 2 N Natronlauge zersetzt, vom gebildeten Natriumaluminat abgesaugt und das Filtrat eingeengt. Der Einengungsrückstand wird aus Acetonitril umkristallisiert.
Ausbeute: 890 mg (27 % der Theorie),
Schmelzpunkt: 103-104° C

| | | | |
|---|---|---|---|
| Ber.: | C 79,74 | H 7,53 | N 3,87 |
| Gef.: | 80,03 | 7,34 | 3,86 |

Beispiel 5

4-[2-[N-(2-Hydroxy-2-(3-trifluormethyl-phenyl)ethyl)amino]propyl]diphenylmethan

3,8 g (20 mMol 3-Trifluormethyl-acetophenon werden in 70 ml Dioxan und 3 ml Wasser gelöst und nach Zugabe von 2 g Kiesel gur und 2,45 g (22 mMol) Selendioxid 6 Stunden am Rückfluß gekocht. Dann wird abgekühlt, filtriert und zum Filtrat, welches das entsprechende Glyoxal enthält, 4,5 g (20 mMol) 4-(2-Amino-propyl)diphenylmethan und 2,6 g (20 mMol) N,N-Diisopropyl-ethylamin gegeben. Nach einer Stunde Rühren bei 35° C wird im Eisbad gekühlt, mit 50 ml Ethanol und zur Reduktion der erhaltenen Schiff'schen Base mit 1 g (26,3 mMol) Natriumborhydrid versetzt. Nach 5 Stunden Rühren bei Raumtemperatur wird eingeengt und der Einengungsrückstand 15 Minuten mit verdünnter Salzsäure gerührt. Dann wird alkalisch gestellt und mit Chloroform extrahiert. Der Chloroformextrakt wird eingeengt und der Einengungsrückstand durch Kieselgelchromatographie gereinigt (Fließmittel: Chloroform/Essigsäure-ethylester/Methanol = 7/2/1).
Ausbeute: 900 mg (11 % der Theorie),
Schmelzpunkt: 104-107° C

| | | | |
|---|---|---|---|
| Ber.: | C 72,62 | H 6,34 | N 3,39 |
| Gef.: | 72,74 | 6,49 | 3,38 |

Laut $^1$H-NMR (400 MHZ) liegt ein 7:1 Diastereomerenpaargemisch vor.
[$CDCl_3$: $\delta$ = 4,64 ppm (dd, 0,87 H), $\delta$ = 4,57 ppm (dd, 0,13 H)]

Analog Beispiel 5 wird folgende Verbindung hergestellt:

a) 4-[2-[N-(2-Hydroxy-2-(3-trifluormethyl-phenyl)ethyl)amino]propyl]biphenyl-hydrochlorid

Hergestellt aus 3-Trifluormethyl-acetophenon und 4-(2-Aminopropyl)biphenyl.
Ausbeute: 11 % der Theorie,
Schmelzpunkt: 188-192° C

| | | | | |
|---|---|---|---|---|
| Ber.: | C 66,12 | H 5,78 | N 3,21 | Cl 8,13 |
| Gef.: | 65,98 | 5,66 | 3,10 | 8,22 |

Laut $^1$H-NMR (400 MHZ) liegt ein 1:5 Diastereomerenpaargemisch vor.
$CDCl_3/CD_3OD$: $\delta$ = 1,32 ppm (d, 2,5 H), $\delta$ = 1,38 ppm (d, 0,5 H)]

Beispiel 6

4′-[2-[N-(2-(3-Chlor-phenyl)-2-hydroxy-ethyl)amino]propyl]biphenylyl-4-carbonsäure

0,5 g (1,1 mMol) 4′-[2-[N-(2-(3-Chlor-phenyl)-2-hydroxyethyl)amino]propyl]biphenylyl-4-carbonsäure-ethylester werden in 30 ml Ethanol suspendiert und nach Zugabe von 2 ml 2 N Natronlauge 5 Stunden bei 50° C gerührt. Anschließend wird mit 4 ml 1 N Salzsäure neutralisiert, filtriert und das Filtrat mit 40 ml Wasser verdünnt. Nach Stehen über Nacht wird vom gebildeten Kristallisat abgesaugt und mit Ethanol/Wasser = 1:1 nachgewaschen.
Ausbeute: 360 mg (80 % der Theorie),
Schmelzpunkt: 199-201° C

| | | | | |
|---|---|---|---|---|
| Ber.: | C 70,32 | H 5,90 | N 3,42 | Cl 8,65 |
| Gef.: | 70,22 | 5,97 | 3,38 | 8,60 |

Laut $^1$H-NMR (400 MHZ) liegt eir 1:1 Diastereomerenpaargemisch vor.
[$DMSO-d_6/CD_3OD$: $\delta$ = 4,98 ppm (dd), $\delta$ = 5,01 ppm (dd)]

Analog Beispiel 6 wird folgende Verbindung hergestellt:

4′-[2-[N-(2-(3-Chlor-phenyl)-2-hydroxy-ethyl)amino]propyl]biphenylyl-4-oxyessigsäure

Hergestellt aus 4′-[2-[N-(2-(3-Chlor-phenyl)-2-hydroxy-ethyl)-amino]propyl]biphenylyl-4-oxyessigsäure-ethylester
Ausbeute: 95,5 % der Theorie,
Schmelzpunkt: 216-218° C

| | | | |
|---|---|---|---|
| Ber.: | C 68,25 | H 5,96 | N 3,18 |
| Gef.: | 68,37 | 5,97 | 3,26 |

Laut $^1$H-NMR (400 MHZ) liegt ein 1:1 Diastereomerenpaargemisch vor.

Beispiel 7

4′-[2-[N-(2-(3-Chlor-phenyl)-2-hydroxy-ethyl)amino]ethyl]biphenylyl-4-carbonsäure-ethylester

1,7 g (10 mMol) 2-(3-Chlor-phenyl)-2-hydroxy-ethylamin und 3,66 g (11 mMol) 4′-(2-Brom-ethyl)-biphenylyl-4-carbonsäureethylester werden in 50 ml Dimethylformamid gelöst und nach Zugabe von 2,8 g (20 mMol) Kaliumcarbonat 3 Stunden bei 90-100° C gerührt. Anschließend wird filtriert, eingeengt und der Einengungsrückstand an Kieselgel säulenchromatographisch gereinigt (Fließmittel: Essigsäure-ethylester/Methanol = 40:1).
Ausbeute: 1,52 g (36 % der Theorie),
Schmelzpunkt: 120-122° C

| | | | | |
|---|---|---|---|---|
| Ber.: | C 70,83 | H 6,18 | N 3,30 | Cl 8,36 |
| Gef.: | 70,76 | 6,09 | 3,24 | 8,46 |

Analog Beispiel 7 wird folgende Verbindung hergestellt:

a) 4-[2-[N-(2-(3-Chlor-phenyl)-2-hydroxy-ethyl)amino]ethyl]diphenylmethan

Hergestellt aus 2-(3-Chlor-phenyl)-2-hydroxy-ethylamin und 4-(2-Brom-ethyl)diphenylmethan.
Ausbeute: 37 % der Theorie,
Schmelzpunkt: 85-87° C

| | | | | |
|---|---|---|---|---|
| Ber.: | C 75,50 | H 6,61 | N 3,83 | Cl 9,69 |
| Gef.: | 75,63 | 6,72 | 3,69 | 9,57 |

Beispiel 8

4′-[2-[N-(2-(3-Chlor-phenyl)-2-hydroxy-ethyl)-amino]propyl]-4-(2-hydroxy-ethoxy)biphenyl

Zu 1,75 g (46 mMol) Lithiumaluminiumhydrid in 40 ml absolutem Tetrahydrofuran tropft man unter Rühren eine Lösung von 1,75 g (3,85 mMol) 4′-[2-[N-(2-(3-Chlor-phenyl)-2-hydroxyethyl)amino]propyl]-biphenylyl-,1-oxyessigsäure-methylester in 30 ml absolutem Tetrahydrofuran. Anschließend erwärmt man eine Stunde am Rückfluß, zersetzt dann durch tropfenweise Zugabe von 4N Natronlauge und filtriert vom gebildeten Natriumaluminat ab. Das Filtrat wird eingeengt und der Rückstand aus Acetonitril umkristallisiert.
Ausbeute: 660 mg (40,2 % der Theorie),
Schmelzpunkt: 120-126° C

| | | | |
|---|---|---|---|
| Ber.: | C 70,49 | H 6,63 | N 3,29 |
| Gef.: | 70,50 | 6,76 | 3,42 |

Laut $^1$H-NMR (400 MHZ) liegt ein 1:1 Diastereomerenpaargemisch vor. [$CDCl_3$: $\delta$ = 4,56 ppm (dd, 0,5 H), $\delta$ = 4,62 ppm (dd, 0,5 H)]

Beispiel I

Dragée mit 10 mg 4'-[2-[N-(2-(3-Chlor-phenyl)-2-hydroxyethyl)amino]propyl]diphenylmethan-2-carbonsäureethylester

Zusammensetzung:

1 Dragée enthält:

| | | |
|---|---|---|
| (1) | Wirksubstanz | 10,0 mg |
| (2) | Milchzucker | 69,0 mg |
| (3) | Maisstärke | 35,0 mg |
| (4) | Polyvinylpyrrolidon | 5,0 mg |
| (5) | Magnesiumstearat | 1,0 mg |
| | | 120,0 mg |

Herstellung:

(1) + (2) + (3) werden gemischt und mit (4) in einer wäßrigen Lösung befeuchtet. Die feuchte Masse wird durch ein Sieb mit 1,6 mm Maschenweite geschlagen und bei 45°C im Umlufttrockenschrank getrocknet. Das trockene Granulat wird durch ein Sieb mit 1 mm Maschenweite gegeben und mit (5) gemischt. Die fertige Mischung wird zu Dragéekernen verpreßt.

| | |
|---|---|
| Kerngewicht: | 120,0 mg |
| Durchmesser: | 7,0 mm |
| Wölbungsradius: | 6,0 mm |

Die so hergestellten Dragéekerne werden auf bekannte Weise mit einer Schicht überzogen, die im Wesentlichen aus Zucker und Talkum besteht. Diese Schicht kann auch Farbauszüge enthalten. Die fertigen Dragees werden mit Wachs poliert.

| | |
|---|---|
| Dragéegewicht: | 180,0 mg |

Beispiel II

Dragée mit 50 mg 4'-[2-[N-(2-(3-Chlor-phenyl)-2-hydroxyethyl)amino]propyl]-diphenylmethan-2-carbonsäure-ethylester

Zusammensetzung:

1 Dragée enthält:

| | | |
|---|---|---|
| (1) | Wirksubstanz | 50,0 mg |
| (2) | Milchzucker | 110,8 mg |
| (3) | Maisstärke | 50,0 mg |
| (4) | Polyvinylpyrrolidon | 8,0 mg |
| (5) | Magnesiumstearat | 1,2 mg |
| | | 220,0 mg |

Herstellung:

Die Herstellung erfolgt analog Beispiel I.

| | |
|---|---|
| Kerngewicht: | 220,0 mg |
| Durchmesser: | 9,0 mm |
| Wölbungsradius: | 8,0 mm |
| Dragéegewicht: | 300,0 mg |

Beispiel III

Tabletten mit 150 mg 4'-[2-[N-(2-(3-Chlor-phenyl)-2-hydroxyethyl)amino]propyl]-diphenylmethan-2-carbonsäure-ethylester

Zusammensetzung:

1 Tablette enthält:

| | | |
|---|---|---|
| (1) | Wirksubstanz | 150,0 mg |
| (2) | Milchzucker | 86,0 mg |
| (3) | Maisstärke | 50,8 mg |
| (4) | Mikrokristalline Zellulose | 25,0 mg |
| (5) | Polyvinylpyrrolidon | 7,0 mg |
| (6) | Magnesiumstearat | 1,2 mg |
| | | 320,0 mg |

Herstellung:

(1) + (2) + (3) + (4) + (5) werden gemischt und mit Wasser befeuchtet. Die feuchte Masse wird durch ein Sieb mit 1,6 mm Maschenweite geschlagen und bei 45 °C getrocknet. Das trockene Granulat wird

nochmals durch dasselbe Sieb gegeben und mit (6) gemischt. Aus der fertigen Mischung werden Tabletten gepreßt.

| | |
|---|---|
| Tablettengewicht: | 320,0 mg |
| Durchmesser: | 10,0 mm |

Die Tabletten werden mit einer Teilkerbe versehen, um die Halbierung zu ermöglichen.

Beispiel IV

Hartgelatinekapseln zu 100 mg 4′-[2-[N-(2-(3-Chlor-phenyl)-2-hydroxy-ethyl)amino]propyl]-diphenylmethan-2-carbonsäureethylester

Zusammensetzung:

1 Kapsel enthält:
Kapselhülle: Hartgelatinekapseln Größe 3
Kapselinhaltsstoffe:

| | | |
|---|---|---|
| (1) | Wirksubstanz | 100,0 mg |
| (2) | Lactose x $1H_2O$ | 38,0 mg |
| (3) | Maisstärke getrocknet | 60,0 mg |
| (4) | Magnesiumstearat | 2,0 mg |
| | Kapselfüllgewicht: | 200,0 mg |
| (5) | Dest. Wasser | q.s. |

Herstellung:

Ein kleiner Teil Lactose wird ca. 10 %ig in dest. Wasser gelöst (Granulierflüssigkeit). Der Wirkstoff, die restliche Lactose sowie Maisstärke werden gemischt und mit der Granulierflüssigkeit durchfeuchtet. Die Masse wird gesiebt, getrocknet und nach nochmaligem Sieben mit Magnesiumstearat homogen gemischt. Das feinkörnige Granulat wird auf einer geeigneten Maschine in Kapseln abgefüllt.

Beispiel V

Hartgelatinekapseln zu 200 mg 4′-[2-[N-(2-(3-Chlor-phenyl)-2-hydroxy-ethyl)amino]propyl]-diphenylmethan-2-carbonsäureethylester

Zusammensetzung:

Kapselhülle: Hartgelatinekapseln Größe 1
Kapselinhaltsstoffe:

| | | |
|---|---|---|
| (1) | Wirksubstanz | 200,0 mg |
| (2) | Lactose x 1 $H_2O$ | 47,0 mg |
| (3) | Maisstärke getrocknet | 70,0 mg |
| (4) | Magnesiumstearat | 3,0 mg |
| | Kapselfüllgewicht: | 320,0 mg |
| (5) | Dest. Wasser | q.s. |

Herstellung:

Ein kleiner Teil Lactose wird ca. 10 %ig in dest. Wasser gelöst (Granulierflüssigkeit). Der Wirkstoff, die restliche Lactose sowie Maisstärke werden gemischt und mit der Granulierflüssigkeit durchfeuchtet. Die Masse wird gesiebt, getrocknet und nach nochmaligem Sieben mit Magnesiumstearat homogen gemischt. Das feinkörnige Granulat wird auf einer geeigneten Maschine in Kapseln abgefüllt.

Beispiel VI

Alleinfutter II für Mastschweine

| | |
|---|---|
| Gerste | 370 g/kg |
| Weizennachmehl | 200 g/kg |
| Maniokmehl | 135 g/kg |
| Ackerbohnen | 100 g/kg |
| Raps-Extraktionsschrot | 100 g/kg |
| Futterfett | 65 g/kg |
| lysinreiches Mineralfutter für Schweine | 20 g/kg |
| Wirkstoff-Vormischung | 10 g/kg |

Diese Komponenten in den angegebenen Mengen ergeben nach sorgfältigem Mischen 1 kg Futter. Die 10 g Wirkstoffvormischung enthalten z.B. 10 mg Wirkstoff und 9,99 g Maisstärke.

Beispiel VII

Mastfutter II für Broiler

| | |
|---|---|
| Mais | 625 g/kg |
| Sojabohnenmehl | 260 g/kg |
| Fleischmehl | 40 g/kg |
| Futterfett | 25 g/kg |
| Sojaöl | 17 g/kg |
| Bicalciumphosphat | 12 g/kg |
| Calciumcarbonat | 6 g/kg |
| Vitamin-/Mineralstoffmischung | 5 g/kg |
| Wirkstoff-Vormischung | 10 g/kg |

Diese Komponenten in den angegebenen Mengen ergeben nach sorgfältigem Mischen 1 kg Futter.

Die 10 g Wirkstoffvormischung enthalten z.B. 10 mg Wirkstoff und 9,99 g Maisstärke.

Beispiel VIII

Kraftfutter für Rinder

| | |
|---|---|
| Zuckerrübenschnitzel | 600,0 g/kg |
| Maiskleber | 100,0 g/kg |
| Malzkeime | 50,0 g/kg |
| Sojabohnenmehl | 35,0 g/kg |
| Weizen | 110,0 g/kg |
| Melasse | 60,0 g/kg |
| Futterphosphate | 12,0 g/kg |
| Calciumcarbonat | 2,5 g/kg |
| Salz | 5,0 g/kg |
| Mineralstoffe | 10,0 g/kg |
| Vitamin-Vormischung | 5,5 g/kg |
| Wirkstoff-Vormischung | 10,0 g/kg |

Diese Komponenten in den angegebenen Mengen ergeben nach sorgfältigem Mischen 1 kg Futter. Die 10 g Wirkstoffvormischung enthalten z.B. 10 mg Wirkstoff und 9,99 g Maisstärke.

Beispiel IX

Mastfutter für Lämmer

| | |
|---|---|
| Gerste | 690 g/kg |
| Sojabohnenmehl | 100 g/kg |
| Mais | 150 g/kg |
| Melasse | 30 g/kg |
| Vitamin-/Mineralstoffmischung | 20 g/kg |
| Wirkstoffvormischung | 10 g/kg |

Diese Komponenten in den angegebenen Mengen ergeben nach sorgfältigem Mischen 1 kg Futter. Die 10 g Wirkstoffvormischung enthalten z.B. 10 mg Wirkstoff und 9,99 g Maisstärke.

**Ansprüche**

1. Neue Phenylethanolamine der Formel

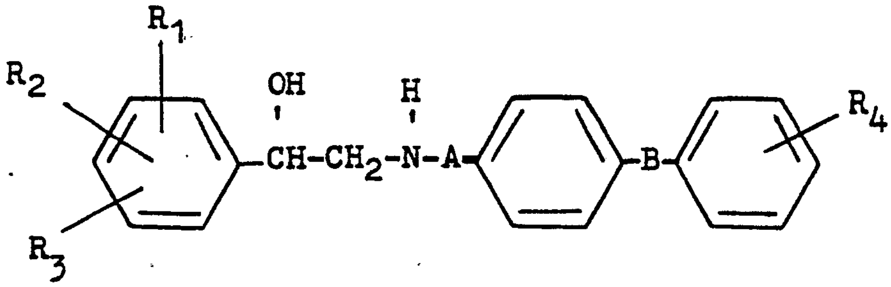

,(I)

in der
A eine geradkettige oder verzweigte Alkylengruppe mit 1 bis 5 Kohlenstoffatomen,
B eine Bindung, eine Alkylengruppe mit 1 oder 2 Kohlenstoffatomen, eine Carbonyl- oder Hydroxymethylengruppe,
$R_1$ ein Wasserstoffatom, ein Halogenatom oder eine Trifluormethylgruppe,
$R_2$ ein Wasserstoffatom oder eine Aminogruppe,
$R_3$ eine Cyanogruppe, ein Wasserstoff-, Chlor- oder Bromatom und
$R_4$ ein Wasserstoff- oder Halogenatom, eine Alkyl-, Hydroxy-, Alkoxy-, Carboxy-, Alkoxycarbonyl-, Aminocarbonyl-, Alkylaminocarbonyl- oder Dialkylaminocarbonylgruppe, eine Alkoxygruppe mit 1 bis 3 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, oder eine Alkoxygruppe mit 2 oder 3 Kohlenstoffatomen, die endständig durch eine Hydroxy-, Alkoxy-, Amino-, Alkylamino-, Dialkylamino-, Pyrrolidino-, Piperidino- oder Hexamethyleniminogruppe substituiert ist, wobei alle vorstehend erwähnten Alkyl- oder Alkoxygruppen, sofern nichts anderes erwähnt wurde, jeweils 1 bis 3 Kohlenstoffatome enthalten können, bedeuten, deren optische Isomere und deren Diastereomere sowie deren Additionssalze.

2. Neue Phenylethanolamine der Formel I gemäß Anspruch 1, in der
A eine gegebenenfalls durch eine Alkylgruppe mit 1 bis 3 Kohlenstoffatomen oder durch zwei Methylgruppen substituierte Ethylengruppe,
B eine Bindung, eine Methylen-, Ethylen-, Hydroxymethylenoder Carbonylgruppe,
$R_1$ ein Wasserstoff-, Fluor-, Chlor- oder Bromatom oder eine Trifluormethylgruppe,
$R_2$ ein Wasserstoffatom oder eine Aminogruppe,
$R_3$ ein Wasserstoffatom, ein Chloratom oder eine Cyanogruppe und
$R_4$ ein Wasserstoffatom, ein Chloratom, eine Hydroxy-, Methoxy-, Methyl-, Ethyl-, Carboxy-, Methoxycarbonyl-, Ethoxycarbonyl-, Carboxymethoxy-, 2-Hydroxy-ethoxy-, Methoxycarbonylmethoxy- oder Ethoxycarbonylmethoxygruppe bedeuten, deren optische Isomere und deren Diastereomere sowie deren Additionssalze.

3. Neue Phenylethanolamine der Formel

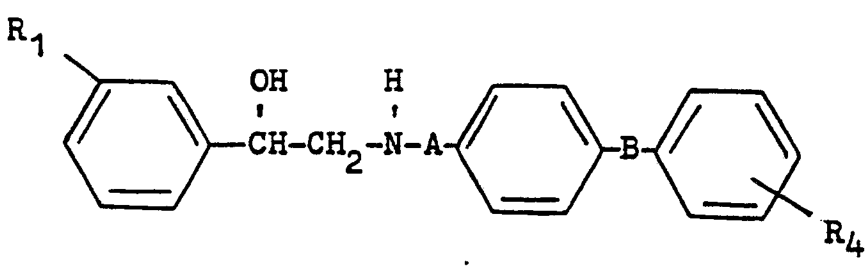

, (Ia)

in der
A eine Ethylen- oder Methyl-ethylengruppe,
B eine Bindung oder eine Methylengruppe,
$R_1$ ein Wasserstoff- oder Chloratom und
$R_4$ ein Wasserstoffatom, eine Methyl-, Ethyl-, Hydroxy-, Methoxy-, Methoxycarbonyl-, Ethoxycarbonyl-, 2-Hydroxy-ethoxy-, Methoxycarbonylmethoxy- oder Ethoxycarbonylmethoxygruppe in 2- oder 4-Stellung bedeuten, deren optische Isomere und deren Diastereomere sowie deren Additionssalze.

4. 4′-[2-[N-(2-(3-Chlor-phenyl)-2-hydroxy-ethyl)amino]propyl]-4-(2-hydroxy-ethoxy)biphenyl, dessen optische Isomere und dessen Diastereomere sowie dessen Säureadditionssalze.

5. 4′-[2-[N-(2-(3-Chlor-phenyl)-2-hydroxy-ethyl)amino]propyl]-diphenylmethan-2-carbonsäure-ethylester,

dessen optische Isomere und dessen Diastereomere sowie dessen Säureadditionssalze.

6. Physiologisch verträgliche Salze der Verbindungen gemäß den Ansprüchen 1 bis 5.

7. Arzneimittel, enthaltend eine Verbindung der Formel I nach mindestens einem der Ansprüche 1 bis 5 oder ein physiologisch verträgliches Additionssalz gemäß Anspruch 6 neben gegebenenfalls einem oder mehreren inerten Trägerstoffen und/oder Verdünnungsmitteln.

8. Verwendung einer Verbindung der Formel I nach mindestens einem der Ansprüche 1 bis 6 zur Herstellung eines Arzneimittels für die Behandlung des Diabetes mellitus, der Adipositas und zur Behandlung und Prophylaxe atherosklerotischer Veränderungen.

9. Verfahren zur Herstellung eines Arzneimittels gemäß Anspruch 7, dadurch gekennzeichnet, daß auf nichtchemischem Wege eine Verbindung der Formel I nach mindestens einem der Ansprüche 1 bis 6 in einen oder mehrere inerte Trägerstoffe und/oder Verdünnungsmittel eingearbeitet wird.

10. Leistungsförderndes Mittel für Tiere, enthaltend eine Verbindung der Formel I nach mindestens einem der Ansprüche 1 bis 6.

11. Verwendung einer Verbindung der Formel I nach mindestens einem der Ansprüche 1 bis 6 als leistungsförderndes Mittel.

12. Tiernahrung und Prämixe zur Herstellung von Tiernahrung, enthaltend eine Verbindung der Formel I nach mindestens einem der Ansprüche 1 bis 6.

13. Verfahren zur Herstellung von leistungsfördernden Mittel für Tiere, dadurch gekennzeichnet, daß eine Verbindung der Formel I nach mindestens einem der Ansprüche 1 bis 6 mit Streck-, Verdünnungs- oder Nahrungsmitteln sowie gegebenenfalls mit weiteren Hilfsstoffen vermischt wird.

14. Verfahren zur Herstellung der neuen Phenylethanolamine gemäß den Ansprüchen 1 bis 6, dadurch gekennzeichnet, daß

a) eine Verbindung der allgemeinen Formel

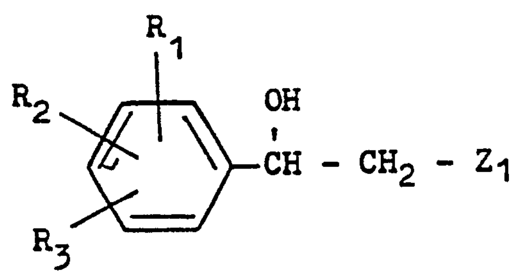

,(II)

mit einer Verbindung der allgemeinen Formel

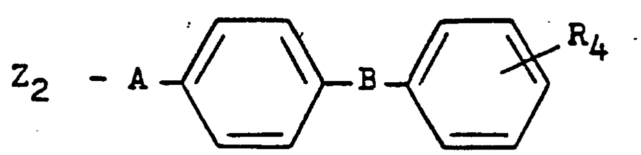

,(III)

in denen

A, B und $R_1$ bis $R_4$ wie in den Ansprüchen 1 bis 5 definiert sind,

einer der Reste $Z_1$ oder $Z_2$ eine nukleophile Austrittsgruppe und

der andere der Reste $Z_1$ oder $Z_2$ eine Aminogruppe bedeuten, umgesetzt wird oder

b) eine gegebenenfalls im Reaktionsgemisch gebildete Schiff'sche Base der allgemeinen Formel

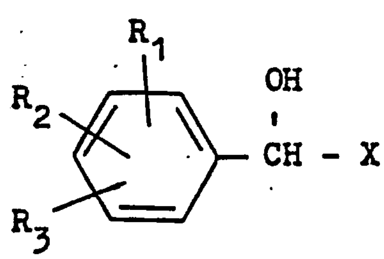

,(IV)

in der
$R_1$ bis $R_3$ wie in den Ansprüchen 1 bis 5 definiert sind und
X eine Gruppe der Formel

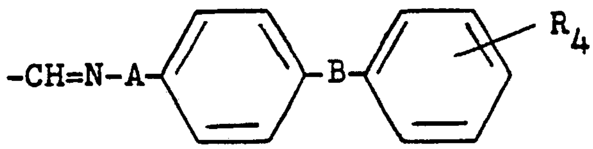

oder

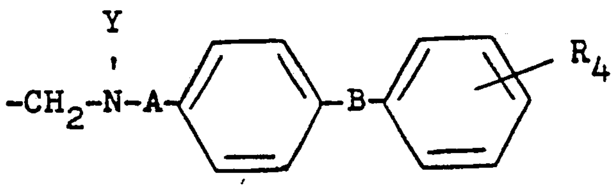

darstellt, wobei
A, B und $R_4$ wie in den Ansprüchen 1 bis 5 definiert sind und Y zusammen mit einem Wasserstoffatom des benachbarten Kohlenstoffatomes des Restes A eine weitere Bindung bedeutet, reduziert wird oder

c) eine gegebenenfalls im Reaktionsgemisch gebildete Verbindung der allgemeinen Formel

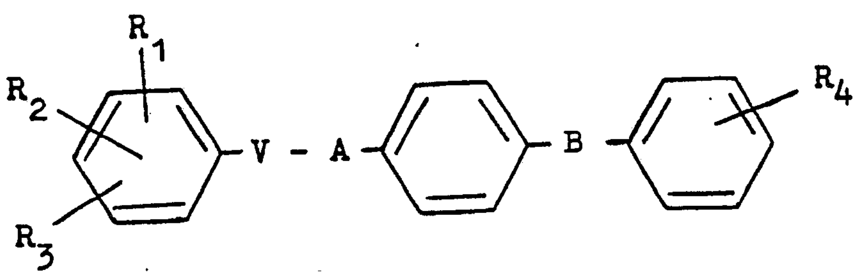

,(V)

in der
A, B und $R_1$ bis $R_4$ wie in den Ansprüchen 1 bis 5 definiert sind und
V eine Gruppe der Formel

$-CO-CH_2-\overset{H}{N}-$ oder
$-CO-CH=N-$ darstellt, reduziert wird oder

d) eine Verbindung der allgemeinen Formel

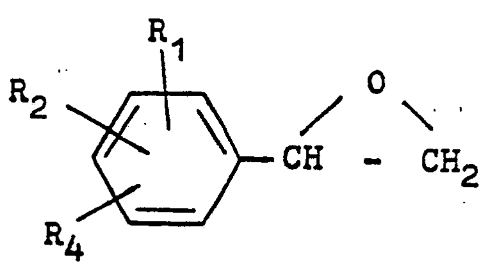

,(VI)

in der
$R_1$ bis $R_3$ wie in den Ansprüchen 1 bis 5 definiert sind, mit einem Amin der allgemeinen Formel

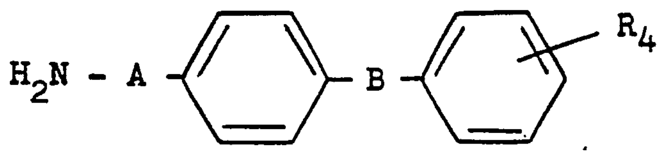

,(VII)

in der
A, B und $R_4$ wie in den Ansprüchen 1 bis 5 definiert sind, umgesetzt wird oder

e) eine gegebenenfalls im Reaktionsgemisch gebildete Verbindung der allgemeinen Formel

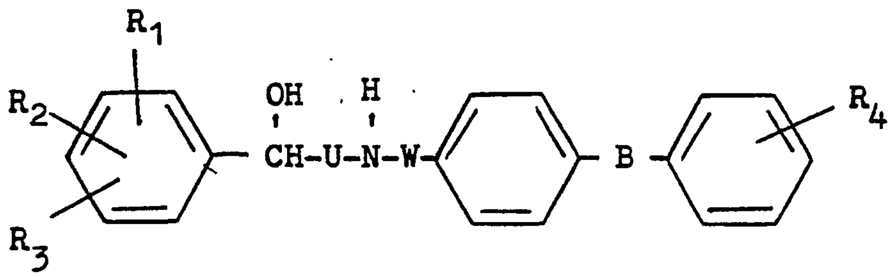

,(VIII)

in der
B und $R_1$ bis $R_4$ wie in den Ansprüchen 1 bis 5 definiert sind,
U eine Carbonylgruppe und W die für A in den Ansprüchen 1 bis 5 erwähnten Bedeutungen besitzt oder
U eine Methylengruppe und W eine Alkanongruppe mit 1 bis 5 Kohlenstoffatomen darstellt, wobei die Carbonylgruppe mit dem Stickstoffatom der NH-Gruppe verknüpft sein muß, reduziert wird oder

f) zur Herstellung von Verbindungen der allgemeinen Formel I, in der $R_3$ ein Wasserstoff-, Chlor- oder Bromatom und $R_4$ eine Alkoxygruppe mit 2 oder 3 Kohlenstoffatomen die endständig durch eine Hydroxy-, Alkoxy-, Amino-, Alkylamino-, Dialkylamino-, Pyrrolidino-, Piperidino- oder Hexamethyleniminogruppe substituiert ist, darstellen, eine Verbindung der allgemeinen Formel

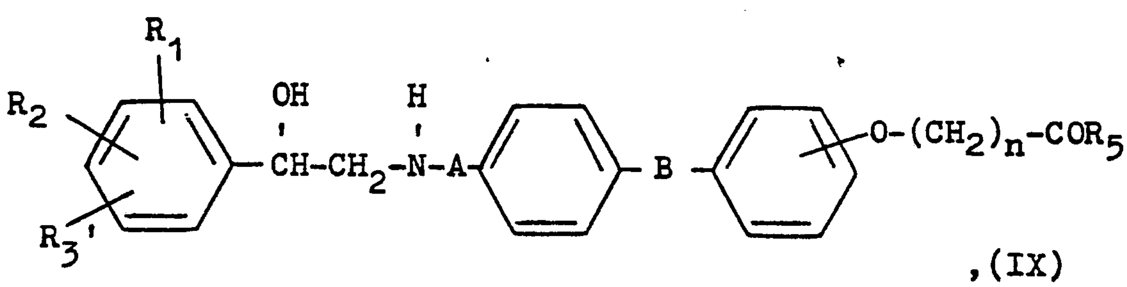

,(IX)

in der
A, B, $R_1$ und $R_2$ wie in den Ansprüchen 1 bis 5 definiert sind,
$R_3'$ ein Wasserstoff-, Chlor- oder Bromatom,
n die Zahl 1 oder 2 und
$R_5$ eine Hydroxy-, Alkoxy-, Amino-, Alkylamino-, Dialkylamino-, Pyrrolidino-, Piperidino- oder Hexamethyleniminogruppe darstellen, reduziert wird oder

g) zur Herstellung von Verbindungen der allgemeinen Formel I, in der $R_4$ eine Alkoxycarbonyl-, Aminocarbonyl-, Alkylaminocarbonyl- oder Dialkylaminocarbonylgruppe, eine Alkoxygruppe mit 1 bis 3 Kohlenstoffatomen, die endständig durch eine Alkoxycarbonyl-, Aminocarbonyl-, Alkylaminocarbonyl- oder Dialkylaminocarbonylgruppe substituiert ist, darstellt, eine Verbindung der allgemeinen Formel

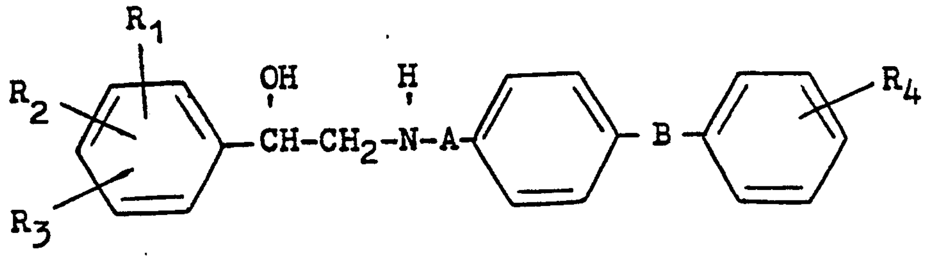

,(X)

in der
A, B und $R_1$ bis $R_3$ wie in den Ansprüchen 1 bis 5 definiert sind und
$R_4'$ eine Carboxygruppe oder eine Alkoxygruppe mit 1 bis 3 Kohlenstoffatomen, welche endständig durch eine Carboxygruppe substituiert ist, darstellt, oder deren reaktionsfähige Derivate mit einer Verbindung der allgemeinen Formel
$H - R_6$ ,(XI)
in der
$R_6$ eine Alkoxy-, Amino-, Alkylamino-, Dialkylamino-, Pyrrolidino-, Piperidino- oder Hexamethyleniminogruppe darstellt, wobei jeweils der Alkyl- oder Alkoxyteil 1 bis 3 Kohlenstoffatome enthalten kann, umgesetzt wird und
erforderlichenfalls anschließend ein bei dem Verfahren a) bis g) verwendeter Schutzrest abgespalten wird und/oder
gewünschtenfalls anschließend eine so erfindungsgemäß erhaltene Verbindung der Formel I, in der $R_4$ eine Alkoxycarbonylgruppe oder eine der in den Ansprüchen 1 bis 5 erwähnten Amidocarbonylgruppen darstellt oder enthält, mittels Hydrolyse in eine entsprechende Verbindung der allgemeinen Formel I, in der $R_4$ eine Carboxygruppe darstellt oder enthält, übergeführt wird und/oder
eine so erhaltene Verbindung der allgemeinen Formel I, in der $R_4$ eine der in den Ansprüchen 1 bis 5 erwähnten Alkoxygruppen darstellt, mittels Etherspaltung in eine entsprechende Verbindung der allgemeinen Formel I, in der $R_4$ eine Hydroxygruppe darstellt, übergeführt wird und/oder
eine so erhaltene Verbindung der allgemeinen Formel I in ihre optischen Isomeren und Diastereomeren aufgetrennt wird und/oder
eine so erhaltene Verbindung oer allgemeinen Formel I in ihre Additionssalze, insbesondere in ihre physiologisch verträglichen Additionssalze übergeführt wird.

Europäisches Patentamt

# EUROPÄISCHER RECHERCHENBERICHT

Nummer der Anmeldung

EP 88 11 9850

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 233 686 (BEECHAM)<br>* Beispiele 5,18; Ansprüche *<br>--- | 1,7-9 | C 07 C 215/30<br>C 07 C 229/38<br>C 07 C 215/54<br>C 07 C 217/62<br>C 07 C 255/59<br>C 07 C 215/68<br>C 07 C 225/16 |
| A | EP-A-0 026 298 (CYANAMID)<br>* Ansprüche *<br>--- | 1,7-10,<br>12 | |
| A | EP-A-0 070 134 (BEECHAM)<br>* Beispiele; Ansprüche *<br>--- | 1,7-9,<br>14 | |
| A | EP-A-0 101 069 (HOFFMANN LA ROCHE)<br>* Ansprüche *<br>--- | 1,7-9,<br>14 | |
| P,X | DE-A-3 718 638 (DR. KARL THOMAE)<br>* Insgesamt *<br>----- | 1-14 | |
| | | | RECHERCHIERTE SACHGEBIETE (Int. Cl.5) |
| | | | C 07 C<br>A 61 K |

Der vorliegende Recherchenbericht wurde für alle Patentansprüche erstellt

| Recherchenort | Abschlußdatum der Recherche | Prüfer |
|---|---|---|
| DEN HAAG | 29-07-1989 | HELPS I.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)